# EUROPEAN PATENT SPECIFICATION

(11) **EP 0 873 397 B1**
(45) Date of publication and mention of the grant of the patent: **04.05.2005**
(21) Application number: 97935822.3
(22) Date of filing: 19.08.1997
(51) Int. Cl.: C12N 1/20

(54) **NOVEL MICROORGANISM AND METHOD FOR ENVIRONMENTAL PURIFICATION USING THE SAME**
NEUER MIKROORGANISMUS UND SEINE BENUTZUNG IN EINER METHODE ZUR UMWELTREINIGUNG
NOUVEAU MICRO-ORGANISME ET PROCEDE DE PURIFICATION DE L'ENVIRONNEMENT UTILISANT CE MICRO-ORGANISME

(30) Priority: 19.08.1996 JP 21745696; 25.04.1997 JP 10955397
(43) Date of publication of application: 28.10.1998
(73) Proprietor: Toyota Jidosha Kabushiki Kaisha, Toyota-shi, Aichi-ken 471-71 (JP)
(72) Inventor: NUMATA, Koichi, Toyota-shi, Aichi 471-71 (JP); ODA, Yasushi, Toyota-shi, Aichi 471-71 (JP); MIYATA, Masami, Toyota-shi, Aichi 471-71 (JP); OKAMURA, Yukio, Toyota-shi, Aichi 471-71 (JP); KIMURA, Toshiaki, Toyota-shi, Aichi 471-71 (JP); UCHIDA, Masatoshi, Toyota-shi, Aichi 471-71 (JP); ASAMI, Osamu, Konan-si, Aichi 483 (JP)
(74) Representative: Leson, Thomas Johannes Alois, Dipl.-Ing.
(86) International application number: PCT/JP1997/002872
(87) International publication number: WO 1998/007831

(56) References cited:
- EP-A- 0 523 769
- DE-A- 19 647 847
- CHEMICAL ABSTRACTS, vol. 124, no. 9, 26 February 1996 Columbus, Ohio, US; abstract no. 109235, PERRY, J. J. ET AL: "Biodegradation of environmental pollutants by Mycobacterium vaccae" XP002051929 & BIODETERIOR. BIODEGRAD. 9, INT. BIODETERIOR. BIODEGRAD. SYMP., [PROC.], 9TH (1995), MEETING DATE 1993, 445-9. EDITOR(S): BOUSHER, ANDREW;CHANDRA, MALINI; EDYVEAN, ROBERT. PUBLISHER: INSTITUTION OF CHEMICAL ENGINEERS, RUGBY, UK. CODEN: 62EXA9,
- CHEMICAL ABSTRACTS, vol. 122, no. 22, 29 May 1995 Columbus, Ohio, US; abstract no. 273088, WILCOX, DONNELL W. ET AL: "Propane-induced biodegradation of vapor phase trichloroethylene" XP002051930 & BIOTECHNOL. BIOENG. (1995), 46(4), 333-42 CODEN: BIBIAU;ISSN: 0006-3592,
- CHEMICAL ABSTRACTS, vol. 122, no. 21, 22 May 1995 Columbus, Ohio, US; abstract no. 260790, VANDERBERG, LAURA A. ET AL: "Biodegradation of trichloroethylene by Mycobacterium vaccae" XP002051931 & CAN. J. MICROBIOL. (1995), 41(3), 298-301 CODEN: CJMIAZ;ISSN: 0008-4166,
- CHEMICAL ABSTRACTS, vol. 91, no. 11, 10 September 1979 Columbus, Ohio, US; abstract no. 87180, SINGH, GAJENDRA ET AL: "On the location, transmission and identification of the symbiotes of Cletus signatus Walker" XP002051932 & INDIAN J. ENTOMOL. (1977), 38(1), 38-46 CODEN: IJENA8;ISSN: 0367-8288,

## Description

### FIELD OF INVENTION

The present invention relates to new microorganisms which efficiently decompose organic halogenated compounds such as trichloroethylene, and to a method for decomposing organic halogenated compounds, specifically trichloroethylene in the soil, the underground water, or waste waters.

### BACKGROUND OF INVENTION

In recent years, industrial utilization of organic solvents has been producing environmental polution problems by discharge of these compounds or waste waters containing these compounds in many parts of the nation. In particular, soil polution by organic chlorinated compounds is a major social problem, and the technology of repairing the contaminated soil has become more essential. The purification method of the contaminated soil includes physical methods and biological methods.

The physical treatment methods include the air-stripping method (a method of purging air into the contaminated soil which was excavated in order to volatize organic chlorinated compounds contained therein, and of removing them by adsorption on activated charcoal), and vacuum extraction methods (a method in which pipes are driven into the contaminated soil to create the state of reduced pressure so that the organic chlorinated compounds therein are volatized and extracted from the soil). However, these methods require an enormous power for purging the air etc., and have the drawbacks that the former methods require excavation of the soil, while in the latter the extraction efficiency is low and purification does not proceed smoothly under the low concentration of the contaminats. Furthermore, both methods only absorb the contaminating substances to the activated charcoal and therefore require a separate means to detoxicate the contaminating substances.

It has been reported recently that the biological treatment method which is under development utilizes the ability of microorganisms to decompose substances and can completely decompose or detoxicate the contaminating substances, and besides less energy is needed for the treatment as compared with the physical means. Moreover, the biological means permits purification even at low concentrations of contaminants and accordingly expectations on the method are great as a low-cost method for soil purification. The known biological methods include the solid-phase treatment (the excavated soil is mixed with phosphorus, nitrogen, microorganisms etc. to promote decomposition of the contaminating substances by the microorganisms), the slurry treatment method (the excavated soil is mixed with water, phosphorus, nitrogen, microorganisms etc. to treat in the liquid form to promote purification speed of the contaminating substances by the microorganisms), and the on-site treatment method (methane, the air, phosphorus, and nitrogen are injected into the soil without excavating the soil to promote decomposition of the contaminating substances by the microorganisms).

Of the conventionally used biological treatment methods, the solid-phase treatment method and the slurry treatment method require excavation of the soil and besides have a narrow range of application, and the cost for treatment and equipment is relatively high.

On the other hand, the on-site treatment method in which indegenous microorganisms are performed as degraders is less expensive in treatment and equipment compared with the methods described above, and can be applied on a wider range. But, under the condition that the absolute number of microorganisms in the soil is small, the purification rate of the on-site treatment decreases. Especially in the case of the compounds refractory to decomposition such as organic chlorinated compounds, purification is impossible when there are no living microorganisms which can decompose said contaminants in the soil. In such cases, it is believed that inoculation of the microorganisms having the ability of decomposing organic chlorinated compounds into the soil is essential for enhancement of the purification late of soil.

Known microorganisms which decompose trichloroethylene include Methylosinus tricosporium OB3 (Japanese Unexamined Patent Publication (Kohyo) No. 4(1992)-501667, Japanese Unexamined Patent Publication No. 5(1993)-212371), and Methylosinus tricosgorium TUKUBA (Japanese Unexamined Patent Publication No. 2(1990)-92274, Japanese Unexamined Patent Publication No. 3(1991)-292970) which are methane-degradatating organisms, Pseudomonas putida F1 (Japanese Unexamined Patent Publication No. 64(1989)-34499), Pseudomonas putida BH (Fujita et al.; Chemical Engineering, 39(6):494-498, 1994), Pseudomonas putida UC-R5, UC-P2 (Japanese Unexamined Patent Publication No. 62(1987)-84780), Pseudomonas putida KWI-9 (Japanese Unexamined Patent Publication No. 6(1994)-70753), Pseudomonas mendocina KR1 (Japanese Unexamined Patent Publication No. 2(1990)-503866, 5(1993)-502593), Pseudomonas cepacia G4 (Japanese Unexamined Patent Publication No. 4(1992)-502277), and Pseudomonas cepacia KK01 (Japanese Unexamined Patent Publication No. 6(1994)-296711) which belong to the genus Pseudomonas, Alcaligenes eutropus JMP134 (A.R. Harker, Appl. Environ. Microbiol., 56(4):1179-1181, 1990), Alcaligenes eutropus KS01 (Japanese Unexamined Patent Publication No. 7(1995)-123976), Nitrosomonas europaea (D. Arciero et al., Biochem. Biophys. Res. Commun., 159(2):640-643, 1989) which is an ammonia-oxidizing bacterium, Corynebacterium J1 (Japanese Unexamined Patent Publication No. 8(1996)-66182) and the like.

The trichloroethylene-decomposing ability of these known microorganisms is not very high and most of these microorganisms can decompose 5 ppm of trichloroethylene in the liquid culture only. Furthermore, since decomposing ability of trichloroethylene in a special environment as the soil is required, it is necessary that the microorganism to be used for bioremediation not only has a sufficient ability of decomposing trichloroethylene but also can remain decomposing ability of trichloroethylene even in the soil. However, most of the known microorganisms are insufficient in this respect.

It is reported that Pseudomonas cepacia KK01 can decompose trichloroethylene at an initial concentration of 30 ppm to 15 ppm in the liquid culture, and trichloroethylene at an initial concentration of 5 ppm to 1 ppm in the soil (Japanese Unexamined Patent Publication No. 6(1994)-296711). Furthermore, it is reported that Alcaligenes eutropus KS01 can decompose trichloroethylene at an initial concentration of 50 ppm to below the level of detection in the liquid culture, and trichloroethylene at an initial concentration of 1 ppm to below the level of detection limit in the soil (Japanese Unexamined Patent Publication No. 7(1995)-123976).

It has been confirmed that these microorganisms have a higher decomposing ability than the conventional microorganisms and that these abilities can be exhibited even in the soil. However, addition of at least one or more than one aromatic compound is needed to the soil environment for induction of the decomposing abilities of these microorganisms. But, the aromatic compounds themselves are contaminants and therefore have a risk of causing a secondary pollution. It is a great challenge to be solved for practical application, therefore, to obtain a microorganism which enables an aromatic compound, when added, to be completely decomposed and removed together with trichloroethylene, or which permits decomposition of trichloroethylene without addition of an aromatic compound.

Accordingly, in order to put the biological purification of trichloroethylene into practical use, it has been desired to obtain a microorganism which has a high decomposing ability, and which enables an aromatic compound, when added, to be completely decomposed and removed together with trichloroethylene, or which permits decomposition of trichloroethylene without addition of an aromatic compound.

Furthermore, in many cases it is extremely difficult to increase the density of a microorganism to the level commensurate with its desired treatment capacity, because the density of the dispersed microorganism is suppressed low in the soil because of predation thereof by protozoa and competitive effects by autochthonous microorganism. In order to increase the density methods are employed such as the method of pressure pumping the air and nutrients into the soil. But despite the enormous energy required, it is difficult to increase the bacterial density by those means alone, thereby keeping the decomposing ability of microorganisms at low levels. Tremendous amounts of energy such as supply of nutrients, aeration etc. are needed to retain a high bacterial density in the closed system such as the reactor as well as in the open system.

If decomposition capability per unit amount of a bacterial mass is increased, a sufficient decomposition capability may be obtained even at low densities of the bacterial mass, thus obviating the need to put in a tremendous amount of energy for keeping the density of the bacterial mass. Though microorganisms which decompose trichloroethylene do so by expressing the enzyme capable of decomposing such substances, such expression of the enzyme requires an inducer. It has already been known that microorganisms can be allowed to exhibit their decomposition ability by adding an inducer and bringing the microorganisms into contact said inducer during culturing, but no previous studies have focused on the length of time of the contact and thereby on the methods to enhance the decomposition ability per unit amount of bacterial mass.

Bio-augmentation which spreading trichloroethylene-decomposing microorganisms into the soil to effect decomposition of trichloroethylene etc. is currently hard to get social acceptance, since it has a potential risk of producing far-reaching effects on the ecological system by releasing a specific microorganism into the environment. But the spraying of a microorganism which has completely lost the propagating activity by sterilization treatment is equivalent to that of mere organic materials, and thus is believed to have little effect on the ecological system. The invention which was disclosed in Japanese post-examined Patent Publication No. 8(1996)-3012 claims that undesirable effects on the ecological system can be minimized by crushing the decomposing bacteria and then spraying them to the soil. But, it will be readily appreciated that the crushing procedure of microorganisms takes extensive equipment, a lot of time and labor, and thus the spraying of a large amount of decomposing bacterium to the contaminated soil will be in fact very difficult.

The above invention further lists the advantageous effects by claiming that the crushed bacteria are easier to penetrate into the soil than the intact bacterial mass, but said invention makes no mention of duration of the decomposing ability retained by the crushed bacteria. Moreover, the known trichloroethylene-oxidase requires NAD as a coenzyme. But it would be extremely difficult to supply the coenzyme in the concentration necessary for the decomposition reaction of the enzyme which is released from the bacterial mass by crushing the bacterium because the coenzyme is very expensive.

CHEMICAL ABSTRACTS, Vol. 124, No. 9, abstract 109235, discloses a strain of Mycobacterium vaccae (JOB-5), which is able to catabolize several organic compounds e.g. trichlorethylene (TCE).

In CHEMICAL ABSTRACTS, Vol. 122, No. 22, abstract 273088, a method for biodegradation of TCE by using Mycobacterium vaccae JOB-5 is described.

CHEMICAL ABSTRACTS, Vol. 122, No. 21, abstract 260790 is directed to the biodegradation of TCE by Mycobacterium vaccae.

EP-A-0 523 769 discloses several gram-positive alkaliphilic bacteria.

DE-A-196 47 847 is directed to methods and devices for decomposing organic compounds as well as to a method for isolating a microorganism and the respective microorganism.

### DISCLOSURE OF THE INVENTION

Thus, it is an object of the present invention to provide new microorganisms which can decompose organic halogenated compounds such as trichloroethylene in a more efficient manner than the conventionally known microorganisms, and a method for decomposing organic halogenated compounds utilizing said microorganism. It is another object of the present invention to provide an inexpensive method for minimizing the effects on the ecological system after a specific microorganism has been released to the environment.

After intensive studies to solve the above-mentioned problems the inventors have successfully isolated a new microorganism which does not belong to any of the known genera of microorganisms and which has a very high ability of decomposing trichloroethylene as compared with any of the conventionally known microorganisms, and thereby completed the present invention.

Thus, the present invention provides a bacterium which has the following taxonomical properties:

**TABLE 1**

| | |
|---|---|
| Morphology | Coccoid, rod shaped |
| Gram staining | + |
| Spore forming | - |
| Motility | - |
| Relationship to oxygen | aerobic |
| Oxidase test | - |
| Catalase test | + |
| Resistance to acid | - |
| Rod-coccus cycle | + |
| Aerial mycelium forming | - |
| Peptide glycan type of cell wall | meso-diaminopimelic acid |
| Glycolyl test | - (acetyl type) |
| Mycolic acid | - |
| GC content of DNA (mole%) | |
| | 73 (by HPLC) |

and, which can decompose trichloroethylene being the strain MO7 (FERM BP-5624).

The present invention further provides a method for decomposing organic halogenated compounds and/or aromatic compounds, said method comprising allowing said bacterium to act on said organic halogenated compounds and/or aromatic compounds. In this case, an organic halogenated compound is importantly trichloroethylene, and an aromatic compound is preferably phenol.

The present invention further provides a method for decomposing organic halogenated compounds in the soil, waste waters, or other waste products containing organic halogenated compounds, said method comprising adding a culture of said bacterium to said soil, waste waters, or other waste products. An organic halogenated compound whicis important in terms of practical application thereof is trichloroethylene.

The bacterial culture as used herein is preferably a cultured bacterial mass. The cultured bacterial mass may be a living bacterial mass or a sterilized bacterial mass.

The present invention also provides a method for decomposing organic halogenated compounds in the soil, waste waters, or other waste products containing organic halogenated compounds, said method comprising inoculating the bacterium of the present invention to said soil, waste waters, or other waste products, adding an aromatic compound or a degradable carbon source or a mixture thereof to said soil, waste waters, or other waste products, and then culturing said inoculated microorganism. Said aromatic compound as used herein is preferably a phenolic compound, for example phenol, and said degradable carbon source as used herein is preferably a saccharide, for example glucose.

The present invention also provides a decomposition agent of organic halogenated compounds and/or aromatic compounds, said agent comprising said cultured bacterial cells of the bacterium of the present invention. Said cultured bacterial mass may be a living bacterial mass or a sterilized bacterial mass. The sterilization treatment of them is, for example, ultraviolet irradiation. The bacterial mass contained in said decomposition agent is preferably in the form of a dried or freezed bacterial mass from a viewpoint of storage.

### BRIEF EXPLANATION OF THE DRAWINGS

Fig. 1 shows the position of strain MO7 of the present invention in the phylogenetic tree constructed using the NJ (proximal conjugation) method.
Fig. 2 shows the position of strain M07 of the present invention in the phylogenetic tree constructed using the UPGMA (average distance) method.
Fig. 3 is a graph which shows the effect of the initial concentrations of trichloroethylene on the decomposition efficiency, obtained using strain M07 of the present invention.
Fig. 4 is a graph which shows the effect of pH on the decomposition efficiency of trichloroethylene (an initial concentration of 30 ppm), obtained using strain MO7 of the present invention.
Fig. 5 is a graph which shows the effect of the temperature on the decomposition efficiency of trichloroethylene (an initial concentration of 30 ppm), obtained using strain M07 of the present invention.
Fig. 6 is a graph which shows the effect of the initial concentration of trichloroethylene on the decomposition efficiency of trichloroethylene (an initial concentration of 30 ppm), obtained using the dead bacterial cells (sterilized with ultra violet irradiation) of strain MO7 of the present invention.

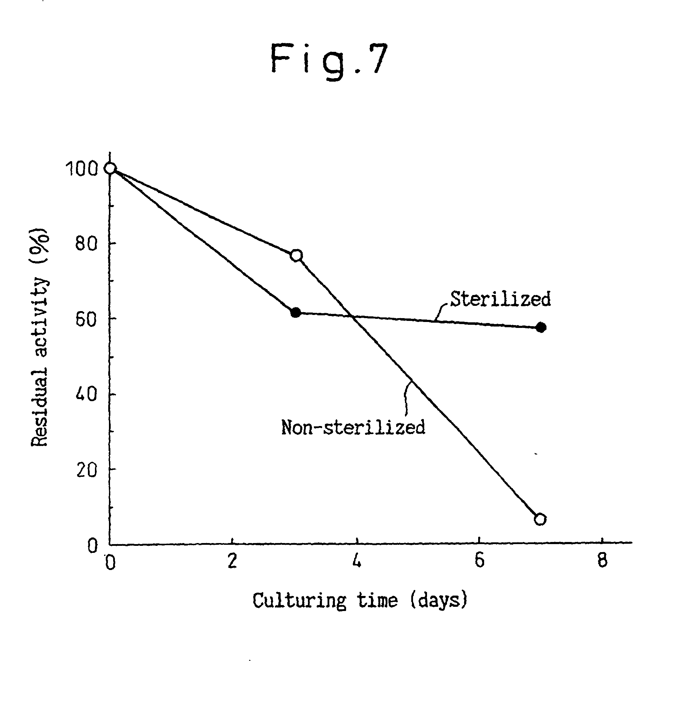

Fig. 7 is a graph which shows a time course of the residual activity of decomposing trichloroethylene using the sterilized (with ultra violet irradiation) and the non-sterilized cultured bacterial cells of strain MO7 of the present invention.
Fig. 8 is a graph which shows growth curve (OD), time course of phenol consumption and the decomposition efficiency of trichloroethylene when strain MO7 of the present invention was cultured in a medium containing phenol and trichloroethylene.
Fig. 9 is a graph which shows growth curve (OD) and a time course of phenol consumption when strain MO7 of the present invention was cultured in a culture medium containing phenol.
Fig. 10 is a graph which shows the specific decomposition efficiency of trichloroethylene per bacterial cells (OD = 1.0) when the bacterium was cultured for various times in the time course of culture as set forth in Fig. 9.
Fig. 11 is a graph which shows the decomposition efficiency of trichloroethylene per total bacterial cells when the bacterium was cultured for various times in the time course of culture as set forth in Fig. 9.
Fig. 12 is a graph which shows growth curve (OD) and a time course of phenol consumption when strain MO7 of the present invention was cultured in a medium containing phenol (500 ppm) and then further cultured by adding phenol when phenol was consumed completely.
Fig. 13 is a graph which shows the specific decomposition efficiency of trichloroethylene per bacterial cells (OD = 1.0) when the bacterium was cultured for various times in the time course of culture as set forth in Fig. 12.
Fig. 14 is a graph which shows the decomposition efficiency of trichloroethylene per total bacterial cells when the bacterium was cultured to various times in the time course of culture of Fig. 12.
Fig. 15 is a graph which shows the relationship between the amount of the bacterial cells and the amount of trichloroethylene decomposed when trichloroethylene was decomposed by adding the cultured bacterial cells of strain MO7 of the present invention into the soil containing trichloroethylene.
Fig. 16 is a graph which shows the amount of trichloroethylene decomposed when the cultured bacterial cells of strain MO7 of the present invention was added at one addition and at two additions.
Fig. 17 is a graph which shows the relationship between the amount of the inoculated bacterial cells and the decomposition efficiency of trichloroethylene at various initial concentration of trichloroethylene in soil when trichloroethylene was decomposed by adding the cultured bacterial mass of strain MO7 of the present invention.
Fig. 18 is a graph which shows a time course of the residual trichloroethylene in soil when trichloroethylene was decomposed by adding the cultured bacterial cells of strain M07 of the present invention to the soil containing trichloroethylene.
Fig. 19 shows a flow chart of the analytical method of vinyl chloride, 1,1-dichloroethylene, cis-1,2-dichloroetylene and trans-1,2-dichloroetylene.
Fig. 20 shows a flow chart of the analytical method of dichloroacetate and trichloroacetate.
Fig. 21 is shows a flow chart of the analytical method of trichloroethanol.
Fig. 22 is shows a flow chart of the analytical method of hydrated chloral.
Fig. 23 is a graph which shows a result when trichloroethylene was decomposed by adding the cultured bacterial cells of strain MO7 of the present invention or the sterilized product thereof to the soil containing trichloroethylene.
Fig. 24 is a graph which shows the effect of temperature (20°C, 30°C) on the decomposition efficiency of trichloroethylene when trichloroethylene was decomposed by adding the cultured bacterial cells of strain MO7 of the present invention to the soil containing trichloroethylene.
Fig. 25 is a graph which shows a result when trichloroethylene was decomposed by adding a small amount of the cultured bacterial cells of strain MO7 of the present invention and adding an inducer (phenol) to the soil containing trichloroethylene.

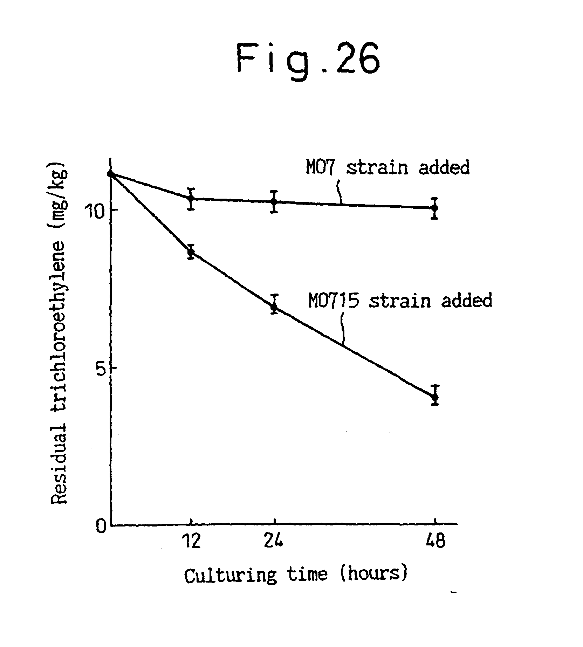

Fig. 26 is a graph which shows a result when trichloroethylene was decomposed by adding the cultured bacterial mass of strain MO7 of the present invention or strain M0715 which were grown on glutamic acid to the soil containing trichloroethylene.

### DETAILED DESCRIPTION

A representative bacterial strain of the present invention, strain MO7 may be isolated in the following manner. For example, an isolation source such as the soil or activated sludge is cultured in a culture medium containing phenol, and the microorganisms which propagated therein are isolated. The isolates are subsequently incubated in a medium containing trichloroethylene to select the microorganisms which have the ability of decomposing trichloroethylene. The method of isolation of microorganisms is described in detail in Example 1.

Strain MO7 thus isolated has the following taxonomical properties:

**TABLE 2**

| | |
|---|---|
| Morphology | Coccoid, rod shaped |
| Gram staining | + |
| Spore forming | - |
| Motility | - |
| Behavior to oxygen | aerobic |
| Oxidase test | - |
| Catalase test | + |
| Resistance to acid | - |
| Rod-coccus cycle | + |
| Aerial mycelium | - |
| Peptide glycan type of cell wall | meso-diaminopimelic acid |
| Glycolyl test | - (acetyl type) |
| Mycolic acid | - |
| GC content of cellular DNA (mole%) | 73 (by HPLC) |

Furthermore, strain MO7 has the following properties.

| | |
|---|---|
| Color of colonies | No characteristic colony color formed |
| Elongation of cells surrounding colony | - |
| Arabinogalactan polymers of cell wall | - (#1) |
| | |

| | |
|---|---|
| #1: Estimated using an acid hydrolysate of the whole bacterial mass | |

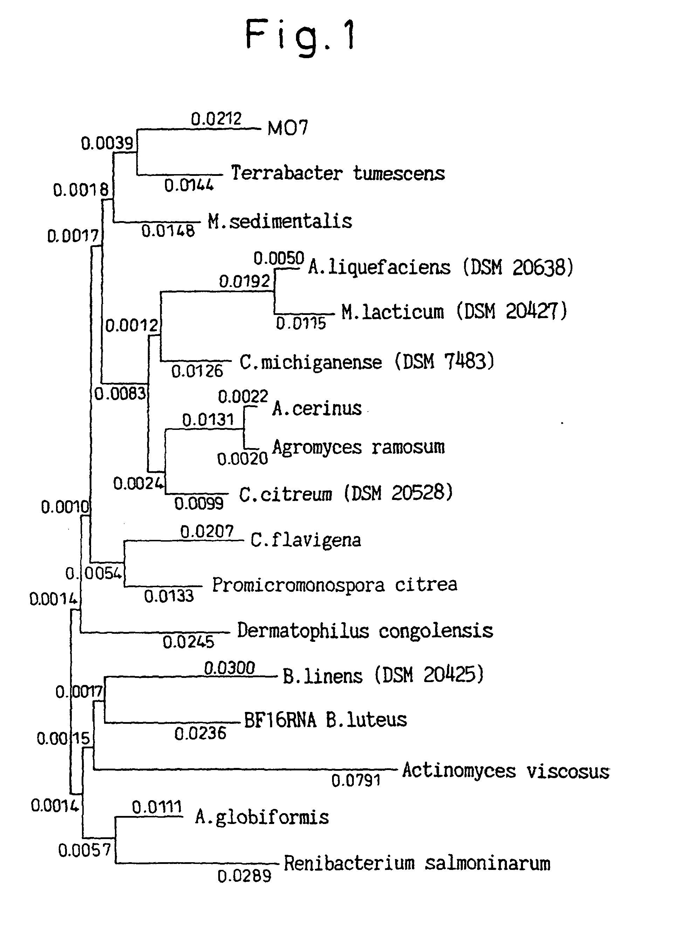

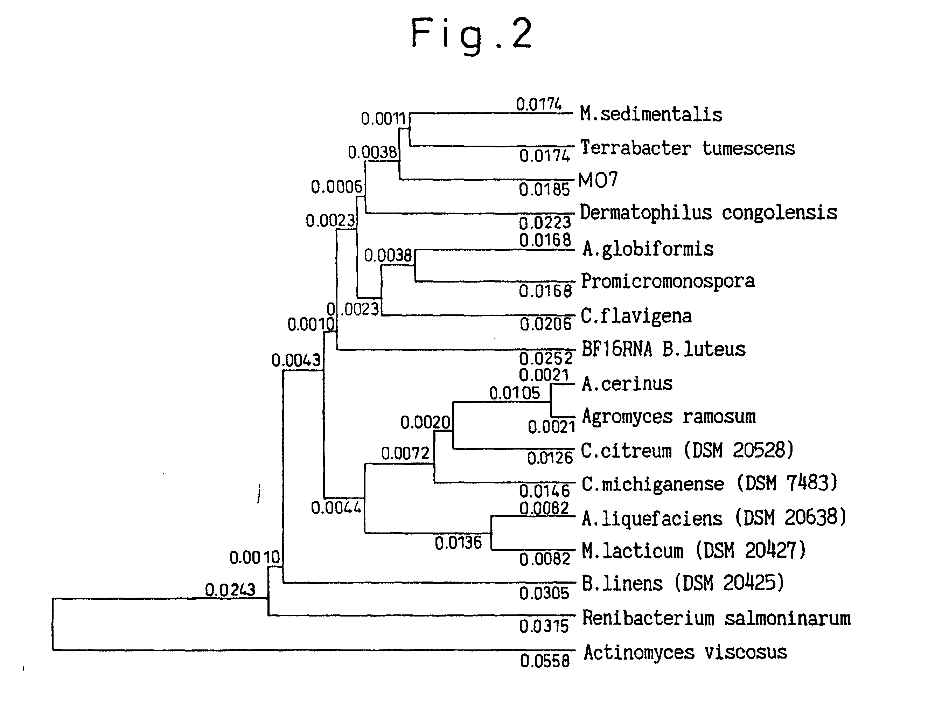

The morphological and physiological characteristics of strain MO7 were investigated and the results obtained are shown in the above Table 2. Based on the results described above identification of the strain was conducted with reference to the publication (N.R. Krieg and J.G. Holt, "Bergy's Manual of Systematic Bacteriology" Vol. 1 (1984), Williams and Wilkins; J.G. Holt, N.R. Krieg, P.H.A. Senath, J.T. Staley and S.T.Williams, "Bergy's Manual of Systematic Bacteriology" Ninthe edition (1984), Williams and Wilkins), with a result that the isolated strain MO7 did not conform to any known genus or species. Also 16S rRNA gene was cloned and its sequence was compared with those of the known organisms to find that the closest relative thereto was Terrabacter tumesces as shown in Fig. 1 and 2. However, even the organism had a homology of 95% at most and thus it was concluded that the isolated strain does not belong to said genus. The bacterial strain of the present invention, therefore, was confirmed to be a new genus which is different from any of the known bacteria.

In the method of the present invention, organic halogenated compounds and/or aromatic compounds are decomposed by allowing the bacterium of the present invention to act on said organic halogenated compounds and/or said aromatic compounds. The organic halogenated compounds decomposed by the bacterium of the present invention include trichloroethylene, dichloroethylene, and vinyl chloride. From a practical point of view, trichloroethylene is most important. As the aromatic compounds there are mentioned phenolic compounds, for example phenol.

In accordance with one embodiment of the present invention, there is provided a method for decomposing organic halogenated compounds in the soil, waste waters, or other waste products, said method comprising adding the culture of the bacterium of the present invention to said soil, waste waters, or other waste products. The bacterial culture as used herein includes the culture liquid itself obtained by culturing the bacterium of the present invention, a cultured bacterial mass isolated from said culture liquid, or a sterilized bacterium in said culture liquid or a sterilized cultured bacterial mass which was isolated.

For culturing of the bacterium of the present invention, any culture medium in which said bacterium can propagate may be used. The culture medium may contain a carbon source such as glucose or sucrose, an organic nitrogen source such as yeast extract, peptone or meat extracts, or an inorganic nitrogen source such as an ammonium salt or nitrate. It may further contain inorganic salts comprising cations such as potassium ion, sodium ion, calcium ion, or magnesium ion, and anions such as chlorine ion, sulfate ion, or phosphate ion. The concentration of the carbon source, though varying depending upon the species, is in the range of about 0.1 to about 1%, and that of the nitrogen source, though varying depending upon the species, is in the range of about 0.01 to about 1%. And that of the inorganic salts, though varying depending upon the species, is in the range of about 0.001 to about 0.1%.

Culturing is preferably conducted by an aerobic liquid culture. The aerobic condition may be secured by a conventional means such as aeration, agitation, aeration and agitation, or shaking. In order to induce the decomposing ability of organic halogenated compounds such as trichloroethylene and that of aromatic compounds such as phenol, an aromatic compound such as phenol is preferably added to the culture medium. In this case, an aromatic compound such as phenol may be added in addition to the other carbon source, or an aromatic compound such as phenol may be added as the only carbon source. The amount of phenol etc. added to the medium is preferably in the range of about 100 ppm to bout 1000 ppm.

In a case where the microorganism of the present invention is used in the form of a bacterial mass, said bacterial mass may be isolated by a conventional means of separating bacterial cells such as centrifugation. When the bacterial mass is stored prior to use, it may be converted into the form of a freezed or dried bacterial mass. In this case, a conventional means for drying a bacterial mass may be employed such as freeze-drying, or spray-drying. In the practice of the present invention, a sterilized culture or a sterilized bacterial mass may be used in order to minimize its effect on the environment (the microbial phase). As a means of sterilization for this purpose, a conventional method such as ultraviolet irradiation to the living bacterial mass may be used. Such sterilized cells or cultures are also encompassed in the "culture" as defined in the present invention.

When the culture of the present invention is added to the subject to be treated, it is preferred to add a living bacterial mass in an amount of 10⁶ to 10⁹ cells/g of the subject to be treated, or a sterilized bacterial mass having the corresponding ability of decomposing organic halogenated compounds.

As a means to allow the microorganism of the present invention to act on an organic halogenated compound such as trichloroethylene, the culture of the present invention need only be added to and mixed with the subject to be treated in order to decompose the organic halogenated compounds contained in the solid such as the soil or the liquid such as waste waters (referred to herein as the subject to be treated).

In accordance with another embodiment of the present invention, organic halogenated compounds such as trichloroethylene contained in the subject to be treated can be decomposed by inoculating the microorganism of the present invention to the subject to be treated such as the soil, waste waters, or other waste products, and then allowing said organism to propagate therein. Thus, the present invention also provides a method for decomposing organic halogenated compounds in the soil, waste waters, or other waste products containing said organic halogenated compounds, said method comprising inoculating the bacterium of the present invention to said soil, waste waters, or other waste products, adding an aromatic compound, an degradable carbon source, or a mixture thereof to the soil, waste waters, or other waste products, and then culturing said inoculated microorganism.

In this case, saccharides, for example glucose, is preferred as the degradable carbon source. The amount of such carbon source is about 0.1 to about 1% relative to the amount of the subject to be treated. Furthermore, when an aromatic compound is added to the subject to be treated, said aromatic compound need to be added. As an aromatic compound, phenol, cresol, and the like may be used. In this case, when the aromatic compound which was added remains after the decomposition of such organic halogenated compounds, it will cause another environmental pollution. Therefore, the addition of an excessive amount of the aromatic compound is not desirable and the amount of the aromatic compound to be added is preferably about 100 to about 500 ppm relative to the subject to be treated.

The inventors have found that the cultured bacterial mass of the present invention prepared as mentioned above, whether it is a living or a sterilized bacterial mass, retains the ability of decomposing organic halogenated compounds and/or aromatic compounds. Thus, the present invention provides a method for decomposing organic halogenated compounds and/or aromatic compounds in the soil, waste waters, or other waste products containing organic halogenated compounds and/or aromatic compounds, in which said sterilized cultured bacterial mass of a microorganism capable of decomposing organic halogenated compounds and/or aromatic compounds is added to said soil, waste waters, or other waste products.

The sterilization methods used includes Ultra violet irradiation, ethylene oxide treatment, radiation, and the like. The sterilized product has an unexpected characteristics of having a higher stability during storage with regard to the ability to decompose organic halogenated compounds.

The present invention also provides a decomposition agent of organic halogenated compounds and/or aromatic compounds, said agent comprising a cultured fo the bacterium of the present invention. The culture is preferably a cultured bacterial mass, which may be a living bacterial mass or a sterilized bacterial mass. Sterilization treatment is carried out by ultra violet irradiation, ethylene oxide treatment, radiation, or another method as described above. From a viewpoint of storage etc. the decomposition agent of the present invention is preferably in the freezed or dried form, which dried product may be obtained according to a conventional method as mentioned above.

The strain MO715, a representative mutant of the present invention (referred to hereinafter as the constitutive mutant) which does not require an aromatic compound (such as phenol) for inducing the activity of trichloroethylene decomposition can be isolated as follows: the strain MO7 is mutated by the action of ultra violet, radiation, or a chemical substance such as nitrosoguanidine which has a mutagenic activity and constitutive mutants are selected from the resulting mutants. The method of isolation of microorganisms is explained in detail in Example 18.

The cultivation of the constitutive mutant of the present invention may be carried out in the same manner as for the parent strain MO7 except that the cultivation of the former does not require an aromatic compound for the induction of the ability of decomposing trichloroethylene. The degradable carbon source for cultivation is preferably a saccharide such as glucose, or an amino acid such as glutamic acid. The method of using it is also the same as for the parent strain MO7 except that the cultivation of the former does not require an aromatic compound for the induction of the ability of decomposing trichloroethylene. It should be noted that the living bacterial mass or the sterilized bacterial mass thereof has the same effect as the parent strain M07 with regard to the ability of decomposing organic halogenated compounds.

The above-mentioned microorganism, the strain MO7, has been internationally deposited on August 12, 1996, with the National Institute of Bioscience and Human-Technology, Agency of Industrial Science and Technology, MITI, as FERM BP-5624 under the provisions of the Budapest Treaty. And the above-mentioned microorganism, the strain MO715, has also been internationally deposited on April 24, 1997, with the National Institute of Bioscience and Human-Technology, Agency of Industrial Science and Technology, MITI, as FERM BP-5928 under the provisions of the Budapest Treaty.

### EXAMPLES

The invention will be understood more readily with reference to the following examples; however these examples are intended to illustrate the invention and are not to be construed to limit the scope of the invention.

### Example 1. Isolation of the MO7 strain as well as cloning and sequencing of 16S rDNA thereof

The microorganisms for use in the present invention were isolated from the activated sludge taken at the waste water treatment plant in Aichi prefecture, Japan, in the following manner. A 0.1 ml aliquot of the harvested activated sludge collected was inoculated into 6 ml of the NMS medium containing 1% (v/v) of a vitamin solution (its composition is shown in Table 5) in a 30 ml vial, to which was added 500 ppm of phenol.

The vial was plugged with a butyl rubber septum and sealed with an aluminum cap, which was then cultured at 30°C under shaking at 160 r.p.m. for a period of from several days to about a dozen days. The culture in which turbidity, even the slightest turbidity, was observed was passaged to the same medium and subsequently cultured under shaking. The passage was repeated for a total of four times. After the fourth passage is over, the culture medium was diluted as appropriate and plated onto the agar plate prepared by adding 1.5% agar to the NYG medium (its composition is shown in Table 5) containing 500 ml phenol. The colonies which appeared were picked onto the agar plate and incubated. This operation was repeated for several times to isolate the microorganisms. In addition to the above NYG medium, another medium such as a nutrient medium can be used after selecting the optimum condition thereof for culturing of microorganisms.

**TABLE 3**

| NMS medium | |
|---|---|
| Magnesium sulfate heptahydrate | 1.0 g |
| Calcium sulfate dihydrate | 0.2 g |
| Potassium nitrate | 0.23 g |
| Ammonium sulfate | 0.65 g |
| Potassium dihydrogen phosphate | 0.272 g |
| Disodium hydrogen phosphate dodecahydrate | 0.727 g |
| Trace Element Solution | 0.5 ml |
| Distilled water | 1 liter |

| The Trace Element Solution | |
|---|---|
| EDTA disodium dihydrate | 500 mg |
| Iron (II) sulfate heptahydrate | 200 mg |
| Zinc sulfate heptahydrate | 10 mg |
| Manganese (II) chloride tetrahydrate | 3 mg |
| Boric acid | 30 mg |
| Cobalt (II) chloride hexahydrate | 20 mg |
| Nickel (II) chloride hexahydrate | 2 mg |
| Sodium molybdenum dihydrate | 3 mg |
| Distilled water | 1 liter |
| * Trace Element Solution was separately sterilized and added after the other ingredients were sterilized. | |

**TABLE 4**

| Vitamin solution | |
|---|---|
| Thiamine hydrochloride | 3 mg |
| p-amino benzoic acid | 13 mg |
| Adenine | 1000 mg |
| NAD | 250 mg |
| Vitamin B12 | 10 mg |
| Thiamine diphosphate | 100 mg |
| Distilled water | 1 liter |

**TABLE 5**

| NYG medium | |
|---|---|
| Yeast Extract | 0.5 g |
| Glucose | 0.18 g |
| NMS medium | 1 liter |

The isolated microorganism was inoculated into 10 ml of the MNS medium containing 0.05% of yeast extract and 500 ppm of phenol in a 18 cm test tube by picking a colony on the agar plate for isolation using a platinum loop. After culturing at 30°C under shaking at 130 r.p.m. for 5 days, the bacterial mass was harvested by centrifugation at 5000 r.p.m. for 10 minutes, and then resuspended into 4 ml of the NMS medium. The suspension of the bacterial mass was placed in a 20 ml vial and trichloroethylene was added in such an amount that it became 30 ppm after all ingredients were dissolved in the liquid phase simultaneously. The vial was plugged with a Teflon-coated butyl rubber septum and sealed with an aluminum cap. After culturing overnight at 30°C, the gas phase in the vial was analyzed by a gaschromatograph equipped with a FID or an ECD detector.

From the results obtained, a bacterial strain having a high ability of decomposing trichloroethylene was selected and characterized for the morphological and physiological properties to obtain the results as shown in Table 2.

Based on said results, identification was carried out with reference to the publication (N.R. Krieg and J.G. Holt, "Bergy's Manual of Systematic Bacteriology" Vol. 1 (1984) Williams and Wilkins, and J.G. Holt, N.R. Krieg, P.H.A. Senath, J.T. Stanley and S.T. Williams, "Bergy's Manual of Determinative Bacteriology" Ninth edition (1984) Williams and Wilkins) to find that this bacterial strain does not belong to any of the known genera or species. The bacterial strain was designated as the MO7 strain and was internationally deposited on August 12, 1996, with the National Institute of Bioscience and Human-Technology, Agency of Industrial Science and Technology, MITI, as FERM BP-5624 under the provisions of the Budapest Treaty.

Furthermore, the sequence of 16S rDNA of the microorganism of the present invention was determined and compared with that of the known microorganisms. The results are outlined hereinbelow.

The 16S rDNA of the microorganism of the present invention was amplified using PCR according to the method of Hiraishi (Journal of Japanese Society for Microbiological Ecology, vol. 10 (1): 31-42, 1995). The primers used for the PCR had the base sequence: 5'-GAG TTT GAT CCT GGC TCA G-3' (SEQ ID No: 1), and 5'-AGA AAG GAG GTG ATC CAG CGG CAG GTT-3' (SEQ ID No: 2). PCR was conducted using a thermal cycler (Perkin Elmer) according to the following program: i.e., preheating at 90°C for 30 seconds, 30 cycles of 96°C for 60 seconds/55°C for 120 seconds/72°C for 180 seconds, and heating at 72°C for 300 seconds.

The amplified PCR fragment was purified using QIAquick (Quiagen) and sequenced by a method which utilized a PCR directly as a template according to the method of Hiraishi (Journal of Japanese Society for Microbiological Ecology, vol. 10 (2): 81-102, 1995). The primers used for the sequence reaction had the base sequence: 5'-GAG TTT GAT CCT GGC TCA G-3' (SEQ ID No: 1), 5'-GGC CGG ACG GGT GAG T-3' (SEQ ID No: 3), 5'-TAC GGG AGG CAG CAG-3' (SEQ ID No: 4), 5'-CTG CCA GCA GCC GCG CG-3' (SEQ ID No: 5), 5'-G ATT AGA TAC CCT GGT AG-3' (SEQ ID No: 6), 5'-ACT CAA AGG AAT TGA CGG-3' (SEQ ID No: 7), 5'-GCA ACG AGC GCA ACC C-3' (SEQ ID No: 8), or 5'-TGT ACA CAC CGC CCG T-3' (SEQ ID No: 9). PCR was conducted using the Dye terminator cycle sequencing kit (Perkin Elmer) according to the protocol instructed in the kit. Sequenced samples were subjected to electrophoresis and sequence analysis using the ABI373A Sequencer (Perkin Elmer). The base sequence determined by these procedures of the 16S rDNA of the microorganism of the present invention is shown in Table 6 (SEQ ID NO:10).

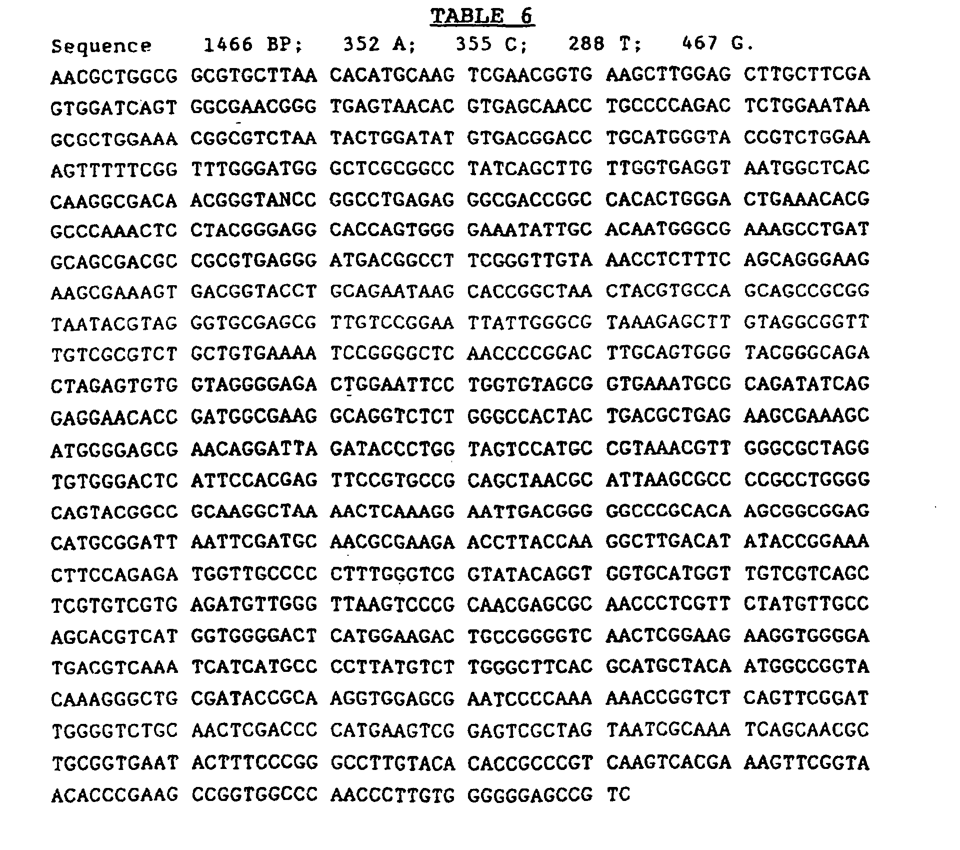

The base sequence thus obtained was compared with the data in DNA database at the National Institute of Heredity (DDBJ+DDBJ NEW). Search of the homologous bacterial strains was conducted using the program "blast" to default value and at first, 50 species were selected which had the highest homology. From among them the bacterial strain (genus Mycobacterium which has the property of producing mycolic acid) which were believed to be definitely different from the results of physiological taxonomic tests were excluded. Then the multiple alignment analysis with the rest of the bacterial strains and the type strains of the genera which are thought to be closely related were conducted.

From the results obtained, a phylogenetic tree was constructed using the NJ (proximal conjugation) method (Fig. 1) or the UPGMA (average distance) method (Fig. 2) (alignment was conducted after the sequences at the 5,-ends were aligned). The most closely related organism turned out to be Terrabacter tumescens (the phylogenic tree includes Mycobacterium sedimentalis for reference). The closely related bacterial strains (microorganisms belonging to the same cluster) were subjected again to homology search using Genetryx-Mac 8.0. But, the homology of the gene of even the closest Terrabacter tumescens was up to 95% and it was judged not to be the same genus. Therefore, it was confirmed that the microorganism of the present invention is a new microorganism different from any of the known microorganism.

The microorganism decomposes about 50% of trichloroethylene at high concentrations of about 100 ppm contaminated in the culture medium, and completely decomposes about 30 ppm of trichloroethylene in 24 hours. In order to decompose trichloroethylene in association with the propagation of the microorganism, it is necessary to add at least one aromatic compound such as phenol in the culture medium (medium, soil, water, etc.) containing trichloroethylene.

The microorganism for use in the present invention will be explained in more detail with reference to the following examples.

### Example 2.

Into 100 ml of the NMS medium containing 0.05% yeast extract and 500 ppm of phenol contained in a 500 ml Erlenmeyer flask was inoculated with a platinum loopful of the colony of the microorganism of the present invention that had been stored by passage on the agar plate of 1.5% agar added to the NYG medium containing 500 ppm phenol, or 1.0 ml of the preculture liquid obtained by culturing overnight under shaking the microorganism of the present invention in the NYG medium containing 500 ppm of phenol at 30°C. After culturing under shaking at 30°C at 130 r.p.m. for 3 days, the bacterial mass was harvested by centrifugation at 5000 r.p.m. for 10 minutes and then resuspended into the NMS medium having an amount equal to the culture medium.

The OD₆₀₀ of the suspension was 0.5 (the cell count was 1 x 10⁹ c.f.u./ml). 4 ml of the suspension of the bacterial mass was dispensed in a 20 ml vial and trichloroethylene was added thereto in such an amount that it became 10, 50, or 30 ppm after all the ingredients were dissolved in the liquid phase. The vial was plugged with a Teflon-coated butyl rubber septum and sealed with an aluminum cap. After culturing overnight at 30°C under shaking, the gas phase in the vial was regularly analyzed by a gaschromatograph equipped with an ECD detector.

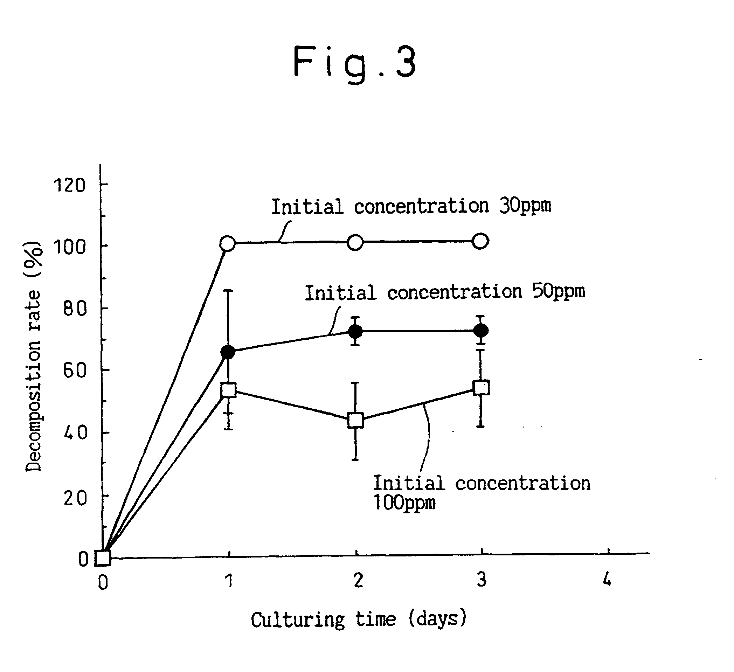

The result is shown in Fig. 3. The present microorganism completely decomposed trichloroethylene in 24 hours. Trichloroethylene at such a high concentration of 50 ppm and 100 ppm was decomposed by 70% and 50%, respectively in 24 hours. Even when the initial concentration of trichloroethylene as high as 50 and 100 ppm, the amount of trichloroethylene decomposed by the present microorganism was not reduced as compared to that when the initial concentration of trichloroethylene was 30 ppm, indicating that the present microorganism is resistant to decomposition inhibition even in the presence of a high concentration of trichloroethylene. Since there has been no reports which demonstrate that as high as 100 ppm of trichloroethylene was decomposed by a microorganism, the decomposition ability of the present microorganism is believed to be extremely high.

### Example 3.

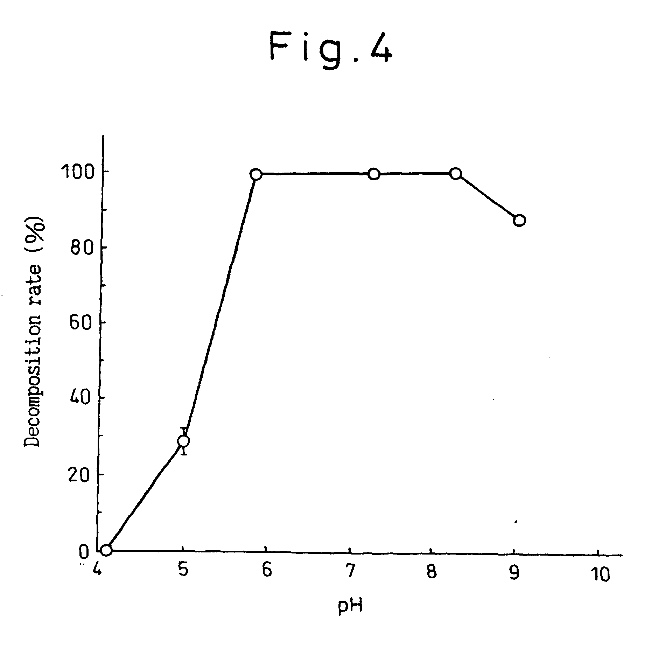

The bacterial mass cultured in the same method as in Example 2 was harvested by centrifugation at 5000 r.p.m. for 10 minutes and then resuspended to each of the M9 medium (Na₂HPO₄·7H₂O 12.8 g/l, KH₂PO₄ 3 g/l, NaCl 0.5 g/l, NH₄Cl 1.0 g/l) prepared in varying pH's at an amount equal to that of the culture liquid. 4 ml of the suspension of the bacterial mass was dispensed in a 20 ml vial and trichloroethylene was added thereto in such an amount that it became 30 ppm after all the ingredients were dissolved in the liquid phase. The vial was plugged with a Teflon-coated butyl rubber septum and sealed with an aluminum cap. After culturing overnight at 30°C under shaking, the gas phase in the vial was regularly analyzed by a gaschromatograph equipped with an ECD detector. As shown in Fig. 4, the result indicated that at pH 5 or lower the activity is extremely decreased, whereas at pH 6 to 9 the efficiency of decomposition was as high as 100% and the activity decreased very slightly to about 90% even at pH 10.

Thus, it turned out that the optimum pH of trichloroethylene decomposition by a resting bacterial mass of the MO7 strain was between 6 and 9, indicating that a high decomposition activity is exhibited at high pH conditions. Trichloroethylene oxide produced by aerobic decomposition of trichloroethylene spontaneously decomposes at an alkaline environment, producing innoxious glyoxylic acid etc. On the other hand, in an acid condition it spontaneously decomposes to produce halo acid. Accordingly, the aerobic decomposition of trichloroethylene by a microorganism is preferably conducted at an alkaline environment. The present microorganism is very useful for decomposition of trichloroethylene at an alkaline environment, since it shows a high decomposition activity at a high pH condition.

### Example 4.

The bacterial mass cultured in the same method as in Example 2 was harvested by centrifugation at 5000 r.p.m. for 10 minutes and then suspended into the same volume of the NMS medium as the culture liquid. 4 ml of the suspension of the bacterial mass was dispensed in a 20 ml vial and trichloroethylene was added thereto in such an amount that it became 30 ppm after all the ingredients were dissolved in the liquid phase. The vial was plugged with a Teflon-coated butyl rubber septum and sealed with an aluminum cap. It was cultured under shaking in an incubator each set at a different temperate. The residual concentration of trichloroethylene was regularly monitored by analyzing the gas phase in the vial with a gaschromatograph equipped with an ECD detector.

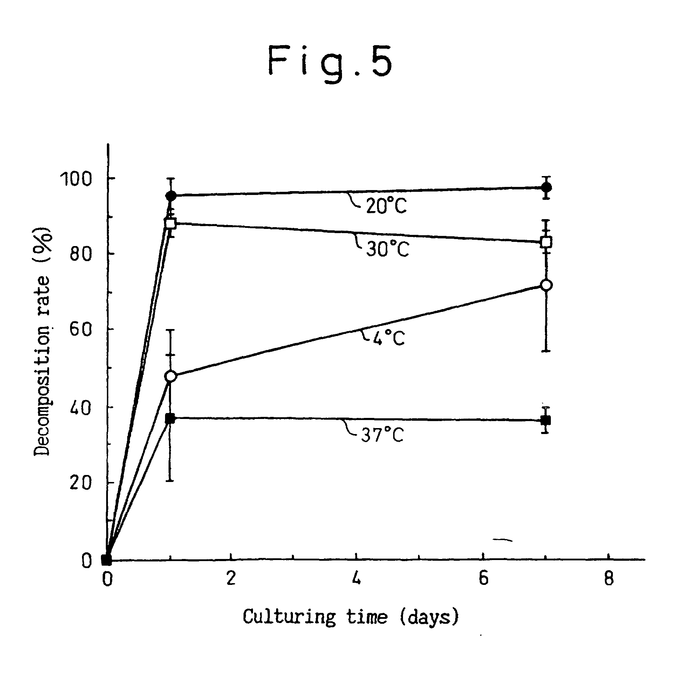

As shown in Fig. 5, the result indicated that the decomposition of trichloroethylene proceeded slowly over 7 days at 4°C. At 37°C, the decomposition ceased in a day and the decomposition efficiency was low. On the other hand, 80 to 90% was decomposed in a day at 20°C and 30°C. But decomposition activity was about 10% higher at 20°C than at 30°C. The temperature of the underground soil where contamination by trichloroethylene etc. is causing a problem is said to be stable at 15 to 20°C. However, in most of the reports on the trichloroethylene-decomposing microorganisms decomposition activity was evaluated at 30°C which is higher than the temperature of the soil, and very few studies have demonstrated that decomposition activity at the same temperature as the soil environment was maintained at a similar level to that at the evaluation experiments.

Since the reaction rate of the enzymatic reaction representing the basic reaction of microbial decomposition usually decreases by one-half with a temperature reduction of 10°C, it is estimated that the decomposition rate of trichloroethylene by a microorganism in the soil having a temperature lower than that at the evaluation experiment is reduced. The present microorganism was demonstrated to have a high practical characteristics without showing reduction in decomposition activity at the same temperature as the soil environment, though the mechanism thereof is unknown.

### Example 5.

The bacterial mass cultured in the same method as in Example 2 was harvested by centrifugation at 5000 r.p.m. for 10 minutes and then suspended to the same volume of the NMS medium as that of the culture liquid. The suspension of the bacterial mass was placed in a petri dish and spread to a thickness of about 1 mm and then sterilized under irradiation of a 15 W ultra violet lamp with a wavelength of 260 nm for not less than 60 seconds at a distance of 40 cm from the light source. 4 ml of the suspension of bacterial mass after sterilization was dispensed in a 20 ml vial and trichloroethylene was added thereto in such an amount that it became 30 ppm after all the ingredients were dissolved in the liquid phase. The vial was plugged with a Teflon-coated butyl rubber septum and sealed with an aluminum cap. It was cultured under shaking at 30°C, and the gas phase in the vial was regularly analyzed with a gaschromatograph equipped with an ECD detector.

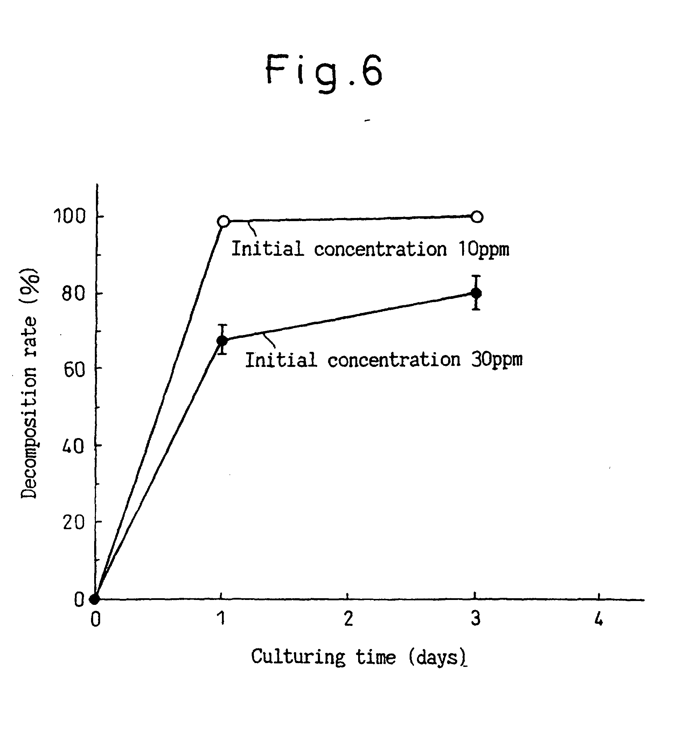

As shown in Fig. 6, the result shows that the bacterial mass after sterilization retains 80% or more of the trichloroethylene-decomposition activity of the living bacterium. Furthermore, in an experiment in which the suspension of sterilized bacterial mass at an amount of 1/100 that of the NYG medium was inoculated or the suspension was plated as it was onto the NYG agar medium and cultured at 30°C, neither increase in turbidity of the culture liquid nor colony formation were observed, and thereby it was confirmed that there was complete sterilization.

The residual activity of the suspension of the sterilized bacterial mass when stored at 4°C was almost the same as that of the living bacterial mass at three days of storage and said suspension had a much higher activity than that of the living bacterial mass at seven days of storage. The results reveal that although the initial activity of the dead bacterial mass is decreased to 80% of the living bacterial mass, a higher activity of decomposing trichloroethylene is retained than the living bacterial mass during storage because of a lower reduction in activity of the dead bacterial mass during storage.

By sterilizing a microorganism without destructing the boundary structure such as the cell wall of a bacterial mass with the extracellular environment, it is possible to retain coenzymes etc. which are required for decomposition reaction of trichloroethylene at concentrations necessary for expression of enzymatic activity. Thus, supply of coenzymes for maintenance of activity of trichloroethylene decomposition is not necessary and the spread of the cultured bacterial mass is only needed. It possible therefore to effect purification of trichloroethylene etc. at low cost and with a minimum effect on the ecological system.

### Example 6.

To 4 ml of the PH medium (see Table 8 for its composition) containing 0.05% yeast extract and 500 ppm of phenol in a 20 ml vial, the present microorganism inoculated by picking a platinum loopful of the colony of the present microorganism from the agar plate prepared by adding 1.5% agar to the NYG medium containing 500 ppm phenol, or by inoculating 0.04 ml (the cell count was 10⁶ cells/ml) of the preculture liquid obtained by culturing the present microorganism in the NYG medium containing 500 m of phenol at 30°C overnight under shaking.

**TABLE 7**

| PH medium | |
|---|---|
| Magnesium sulfate heptahydrate | 0.2 g |
| Calcium sulfate | 0.1 g |
| Ferric chloride hexahydrate | 0.02 g |
| Dipotassium hydrogen phosphate | 1.0 g |
| Ammonium sulfate | 1.0 g |
| Sodium chloride | 0.1 g |
| Distilled water | 1 liter |

Trichloroethylene in such an amount that it became 30 ppm after all ingredients were dissolved in the liquid phase simultaneously with the inoculation and 500 ppm of phenol were added. The vial was plugged with a Teflon-coated butyl rubber septum and sealed with an aluminum cap. It was cultured under shaking at 30°C, and trichloroethylene concentration was regularly measured by analyzing the gas phase in the vial with a gaschromatograph equipped with an ECD detector. The phenol concentration was determined by filtering the culture liquid with a 0.45 µ filter, and adding to 1 ml of the resulting filtrate, sequentially, 0.1 ml of an aqueous solution of K₃Fe(CN)₆/0.1 M glycine and 1 ml of an aqueous solution of 4-amino antipyrine, which was then mixed, and measuring an absorbance at 505 nm.

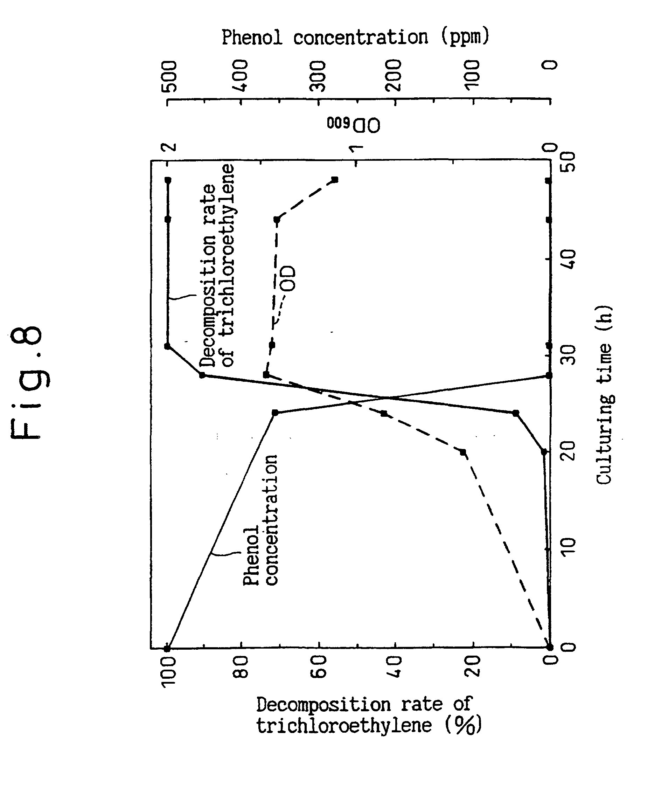

The result as shown in Fig. 8 indicates that with the propagation of the bacterial mass phenol and trichloroethylene decreases, and they were completely decomposed at 31 hours after cultivation. It was confirmed, therefore, that the present microorganism has a high activity of decomposing trichloroethylene when propagated in the presence of an aromatic compound such as phenol having the ability of inducing trichloroethylene. When compared to known microorganisms, all reports relate to decomposition of trichloroethylene at a low concentration of up to 10 ppm except for the report that Pseudomonas cepacia KK01 decomposes 30 ppm of trichloroethylene to 15 ppm in 2 days and the report that Alcaligenes eutropus KS01 (Japanese Unexamined Patent Publication No. 7(1995)-123976) decomposes completely 50 and 25 ppm of trichloroethylene in 4 days.

In the decomposition of trichloroethylene by Alcaligenes eutropus KS01 (Japanese Unexamined Patent Publication No. 7(1995)-123976), the amount of the microorganism inoculated to the culture medium is 10⁸ cells/ml, which is about 100 times that of the present microorganism described in Example 6. The amount of the bacterial mass required for trichloroethylene decomposition for the microorganism of the present invention is much smaller than that of Alcaligenes eutropus KS01. It therefore has an advantage that the cost of culturing is reduced, etc. It was also confirmed that the added and mixed phenol is decomposed to below the level of detection, thereby presenting little risk of environmental pollution by phenol which is an environmental pollutant.

The inventors have focused and investigated on the culturing method prior to inoculation in order to enhance the decomposition activity of a microorganism which has the ability of decomposing trichloroethylene in the soil and thereby to prepare a microorganism having the decomposition activity which can deal with pollution at high concentrations. As a result we have discovered that there is an optimum value of culturing time of the microorganism prior to inoculation, and that the activity of trichloroethylene decomposition can be enhanced by sequentially adding an inducer to the culture medium, and we have completed the present invention. The present invention will now be more fully explained with reference to the following examples.

### Example 7.

Into 100 ml of the PH medium containing 0.05% yeast extract and 500 ppm of phenol contained in a 500 ml Erlenmeyer flask was inoculated a platinum loopful of the colony of the microorganism of the present invention which had been stored by passage on the agar plate of 1.5% agar added to the NYG medium containing 500 ppm phenol, or 1.0 ml of the preculture liquid obtained by culturing the microorganism of the present invention in the NYG medium containing 500 ppm of phenol at 30°C overnight under shaking. While culturing under shaking at 30°C at 130 r.p.m., turbidity of the culture liquid and phenol concentration were determined in the same methods as in Example 6. The bacterial mass in the culture was harvested by centrifugation at 5000 r.p.m. for 10 minutes and then resuspended into the NMS medium having an amount equal to the culture medium. The OD₆₀₀ of the suspension was 0.2 (the cell count was 1 x 10⁸ c.f.u./ml).

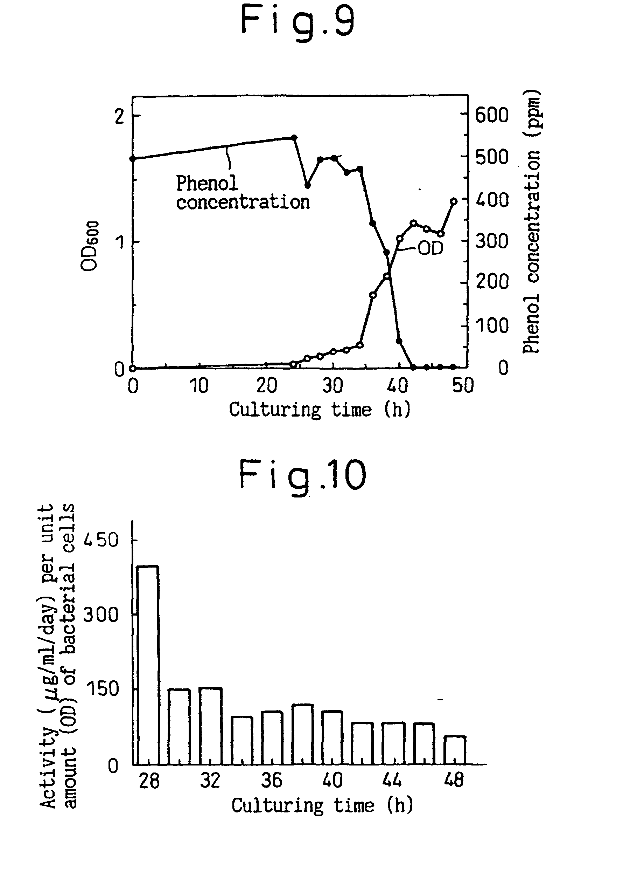

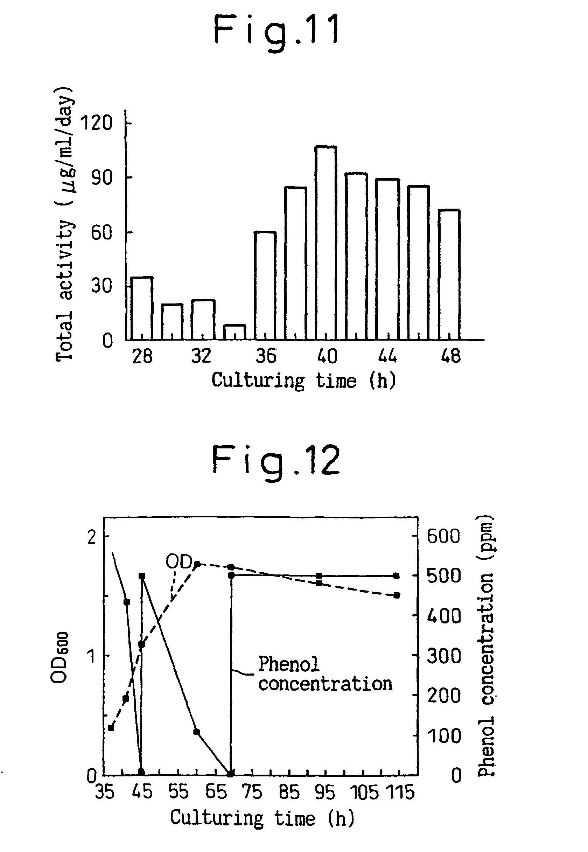

The suspension of the bacterial mass was dispensed in a 20 ml vial and trichloroethylene was added thereto in such an amount that it became 30 ppm when all the ingredients were dissolved in the liquid phase. The vial was plugged with a Teflon-coated butyl rubber septum and sealed with an aluminum cap. After culturing at 30°C under shaking for 24 hours, the gas phase in the vial was analyzed by a gaschromatograph equipped with an ECD detector. The results are shown in Fig. 9 to Fig. 11. The line graphs in Fig. 9 represent turbidity of the culture liquid and phenol concentration. The bar graphs in Fig. 10 represent the decomposition activity per unit amount of the bacterial mass (specific activity) of the microorganism of the present invention harvested at respective time of culturing. The bar graphs in Fig. 11 represent the decomposition activity per unit amount of the culture liquid (the total activity) of the microorganism of the present invention which was harvested at respective time of culturing.

From the results obtained as above on the activity of trichloroethylene decomposition, the specific activity notably increases from the induction phase to the logarithmic growth phase, leveling off thereafter at a certain level. But it gradually decreased after the residual phenol became zero. However, the total activity remained at the highest value at the stationary phase when phenol concentration became around zero. These observations have shown that a bacterial mass having a high activity of trichloroethylene decomposition can be obtained by culturing while monitoring the turbidity of culture liquid and phenol concentration prior to inoculation into the soil, and terminating the culture at the timing that the increase in turbidity ceases to enter the stationary phase and phenol concentration becomes almost zero.

### Example 8.

The microorganism of the present invention was cultured in a manner similar to Example 7 and turbidity of the culture liquid and phenol concentration were measured in a manner similar to Example 6. Furthermore, when the phenol concentration reached zero in the middle of culturing 500 ppm of phenol was added again and culture was continued. The bacterial mass in the culture was harvested by centrifuging at 5000 r.p.m. for 10 minutes and then resuspended into the NMS medium having an amount equal to that of the culture medium.

The OD₆₀₀ of the suspension was 0.2 (the cell count was 2.5 x 10⁸ c.f.u./ml). The suspension of the bacterial mass was dispensed in a 20 ml vial and trichloroethylene was added thereto in such an amount that it became 30 ppm when all the ingredients were dissolved in the liquid phase. The vial was plugged with a Teflon-coated butyl rubber stopper and sealed with an aluminum cap. After culturing at 30°C under shaking for 24 hours, the gas phase in the vial was analyzed by a gaschromatograph equipped with an ECD detector.

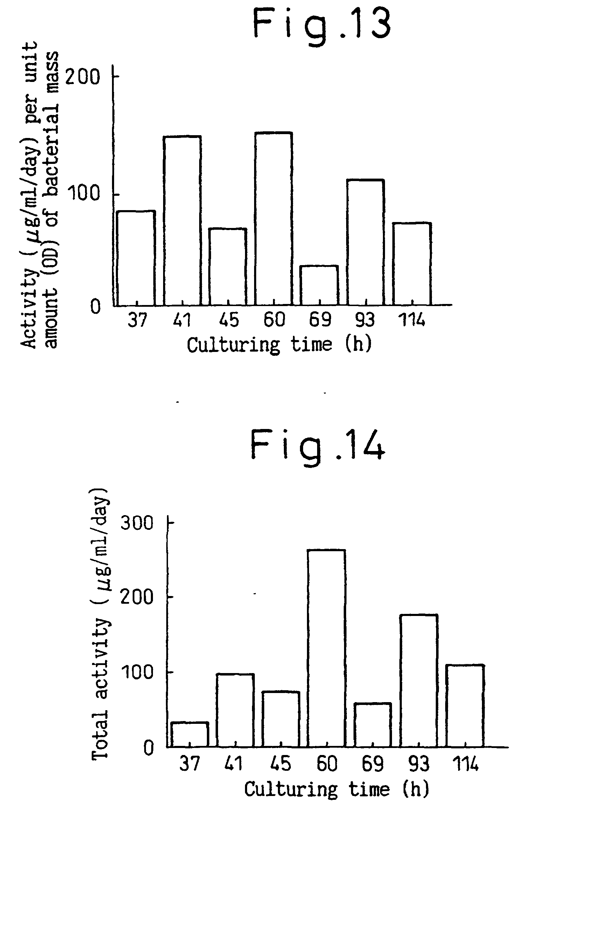

The results are shown in Fig. 12 to Fig. 14. The line graphs in Fig. 12 represent turbidity of the culture liquid and phenol concentration at the stage of culturing prior to harvesting. The bar graphs in Fig. 13 represent the specific activity of the microorganism of the present invention which was harvested at respective time of culturing. The bar graphs in Fig. 14 represent the total activity of the microorganism of the present invention which was harvested at respective time of culturing. When 500 ppm of phenol was added to the culture liquid at 45 hours of culturing when the residual phenol became almost zero, the phenol concentration decreased again and the turbidity of the culture liquid increased. Therefore, phenol was added again at 69 hours, but neither increase in turbidity of the culture liquid nor decrease in phenol concentration were observed.

With regard to the activity of trichloroethylene decomposition, high specific activities were obtained when sufficient phenol is remaining at around the stationary phase during 41 hours to 60 hours as shown in Fig. 13, but the specific activity decreased thereafter when phenol concentration decreased. When phenol was added at 93 hours, both the amount of the bacterial mass and the specific activity decreased. However, during the period from 93 hours to 114 hours when there was residual phenol, the specific activity was higher than during 45 hours to 69 hours when most of the phenol was decomposed. On the other hand, the total activity was the highest at 60 hours when both of the amount of the bacterial mass and specific activity were high, providing the total activity 2.5 times higher than when phenol is added only at the start of culturing.

Next, trichloroethylene decomposition in the soil will be explained with reference to the following examples.

### Example 9.

To a 100 ml vial was added 30 g of sandy soil (air-dried) which was artificially contaminated with trichloroethylene. The microorganism of the present invention was cultured under shaking at 30°C for 3 days in 1.5 ml, 4.5 ml, 7.5 ml, 15 ml, or 30 ml of the NMS medium which had 500 ppm of phenol and 0.05% yeast extract added thereto, and then was centrifuged to harvest the cells, which were resuspended into an appropriate amount of the NMS medium so that the water content in the soil became 25% after addition of the suspension of the bacterial mass. After adding the suspension (containing no phenol) of the bacterial mass to the soil, the vial was sealed with a Teflon-coated rubber septum and sealed with an aluminium cap, and then allowed to stand at 30°C for a certain period of time.

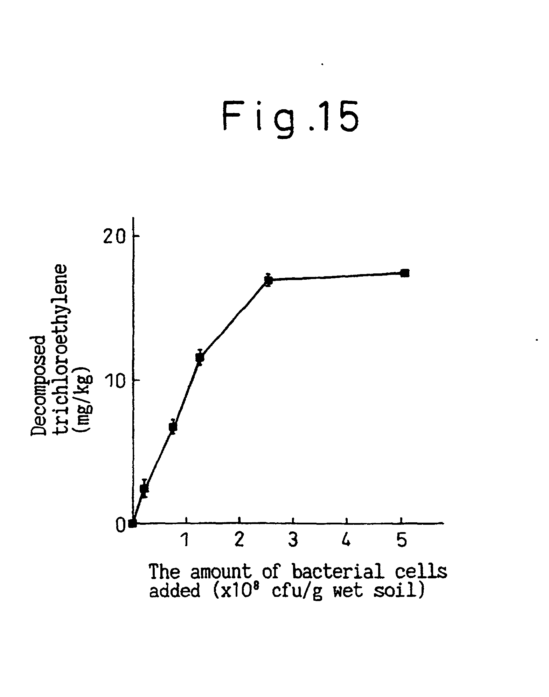

Thereafter, to a vial containing 30 g of the soil were added 50 ml of deionized water aerated with the air which had been passed through activated charcoal and 10 ml of n-hexane, and then the vial was sealed. The vial was sonicated in the sonicating washer for 10 minutes and then was shaken in a shaker for 10 minutes. The separated n-hexane was analyzed using a gaschromatograph equipped with an ECD detector. The result as shown in Fig. 15 indicated that trichloroethylene decomposition and the density of the bacterial mass correlated until the density of the bacterial mass reached 2.5 x 10⁸ cfu/g wet soil (15 ml of the culture liquid), but it became saturated at about 5 x 10⁸ cfu/g wet soil (30 ml of the culture liquid) or higher. Therefore, it was revealed that when the microorganism of the present invention is used to decompose trichloroethylene the density of the bacterial mass of about 2.5 x 10⁸ cfu/g wet soil is most suitable and that the microorganism of the present invention has a characteristics which enables an estimation of a minimum amount of bacterial mass required for purification of a given concentration of a contaminant in the soil.

### Example 10.

To a 100 ml vial was added 30 g of sandy soil (air-dried) which was artificially contaminated with trichloroethylene. The microorganism of the present invention was cultured under shaking at 30°C for 3 days in 7.5 ml of the NMS medium which had 500 ppm of phenol and 0.05% yeast extract added thereto, and then was centrifuged to harvest the cells, which were then resuspended into an appropriate amount of the NMS medium so that the water content in the soil became 25% after addition of the suspension of the bacterial mass. After adding the suspension (containing no phenol) of the bacterial mass to the soil, the vial was sealed with a Teflon-coated rubber septum and sealed with an aluminium cap, and then allowed to stand at 30°C for a certain period of time.

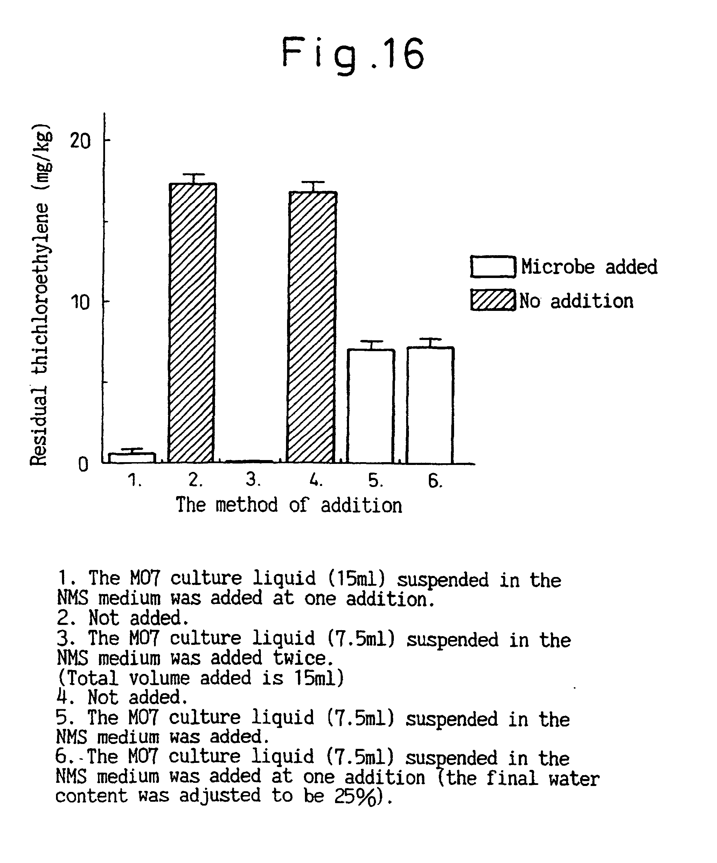

Thereafter, to a vial containing 30 g of the soil were added 50 ml of deionized water aerated with the air which had been passed through activated charcoal and 10 ml of n-hexane, and the vial was sealed. The vial was sonicated in the sonicating washer for 10 minutes and then was shaken in a shaker for 10 minutes. The separated n-hexane was analyzed using a gaschromatograph equipped with an ECD detector. The result as shown in Fig. 16 indicates that when the bacterial mass corresponding to 7.5 ml of the culture liquid was added twice to the soil, the efficiency of decomposition was equal to when the bacterial mass corresponding to 15 ml of the culture liquid was added at one addition.

It is difficult to supply at one addition the amount of culture liquid necessary to decompose trichloroethylene contained in the contaminated soil in terms of the actual amount needed (the actual amount of the contaminated soil is more than a few dozen m³), and the decomposing microorganism must be sequentially cultured and added to the soil. It was revealed that sequential addition of the microorganism of the present invention gives the same effect as that obtained when the required bacterial mass is added at one addition. Therefore, by adding a small amount of the culture liquid repeatedly the microorganism of the present invention can deal with a vast area of the contaminated site for which one-time culture and infusion of the bacterial mass is insufficient.

### Example 11.

To a 100 ml vial was added 30 g of sand grain soil (air-dried) which was artificially contaminated with trichloroethylene at the desired concentrations. The concentration of the contaminant in the soil was set at 15, 30, 45, 100, and 150 mg/kg. The microorganism of the present invention was cultured under shaking at 30°C for 3 days in 15, 30, and.45 ml of the NMS medium which had 500 ppm of phenol and 0.05% yeast extract added thereto, and then was centrifuged to harvest the cells, which were resuspended into an appropriate amount of the NMS medium so that the water content in the soil became 25% after addition of the suspension of the bacterial mass. After adding the suspension (containing no phenol) of the bacterial mass to the soil, the vial was sealed with a Teflon-coated rubber septum and sealed with an aluminium cap, and then allowed to stand at 30°C for a certain period of time. The density of the bacterial mass in the soil 9.4 x 10⁸, 1.9 x 10⁹, and 2.8 x 10⁹ cfu/g wet soil for 15, 30, and 45 ml of the culture liquid, respectively.

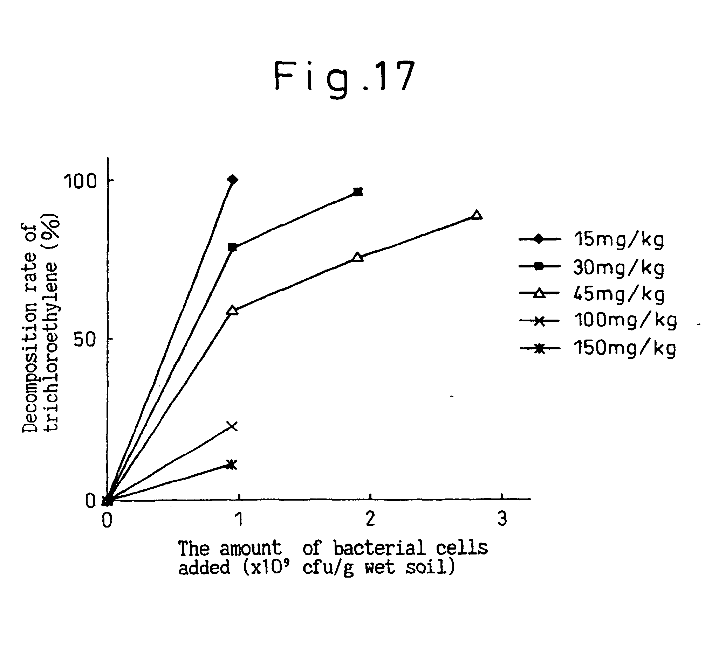

Thereafter, to a vial containing 30 g of the soil were added 50 ml of deionized water aerated with the air which had been passed through activated charcoal and 10 ml of n-hexane, and then the vial was sealed. The vial was sonicated in the sonicating washer for 10 minutes and then was shaken in a shaker for 10 minutes. The separated n-hexane was analyzed using a gaschromatograph equipped with an ECD detector. The result as shown in Fig. 17 indicates that a very high concentration (about 150 mg/kg) of trichloroethylene in the soil could be decomposed. The above result indicated that purification of a high concentration (about 150 mg/kg) contaminant can be effected by increasing the amount of the bacterial mass added or by sequential addition because the amount of trichloroethylene decomposed increases in proportion to the amount of the bacterial mass added to the soil.

### Example 12.

To a 100 ml vial which can be sealed with a Teflon-coated rubber septum was added 30 g of sandy soil (air-dried) which was artificially contaminated with trichloroethylene. The microorganism of the present invention was cultured under shaking at 30°C for 3 days in the NMS medium which had 500 ppm of phenol and 0.05% yeast extract added thereto, and then was centrifuged to harvest the cells, which were resuspended into an appropriate amount of the NMS medium so that the water content in the soil became 25% and the inoculated amount of bacterial mass became 10⁸ to 10⁹ cells/g wet soil after addition of the suspension of the bacterial mass. After adding the suspension (containing no phenol) of the bacterial mass to the soil, the vial was sealed with a Teflon-coated rubber stopper, and then allowed to stand at 30°C for a certain period of time.

Thereafter, to a vial containing 30 g of the soil were added 50 ml of deionized water aerated with the air which was passed through activated charcoal and 10 ml of n-hexane, and the vial was sealed. The vial was sonicated in the sonicating washer for 10 minutes and then was shaken in a shaker for 10 minutes. The separated n-hexane was analyzed using a gaschromatograph equipped with an ECD detector. Time course of changes in trichloroethylene concentration in the soil was determined by preparing a multiple of samples at the same time and extracting the entire volume of a part of samples after passage of a given time, followed by measurement. They were stored at 4°C until extraction.

The measurement of trichloroethylene concentration in the soil was also conducted using a method indicated in the Environmental Standard (the Soil Environmental Standard) related to the contamination of the soil. Thus, the soil sample and the solvent (hydrochloric acid was added to purified water and pH was adjusted to 5.8 to 6.3) were added at a weight to volume ratio of 10% to an Erlenmeyer flask with a screw socket having a stirrer bar, and then the flask was immediately sealed. At this time, care was taken to make the volume of the mixture be not less than 500 ml and to minimize the head space in the Erlenmeyer flask with a screw socket relative to the volume of the mixture. The prepared sample liquid was stirred continuously for 4 hours with the magnetic bar keeping the liquid at ordinary temperature and ordinary pressure.

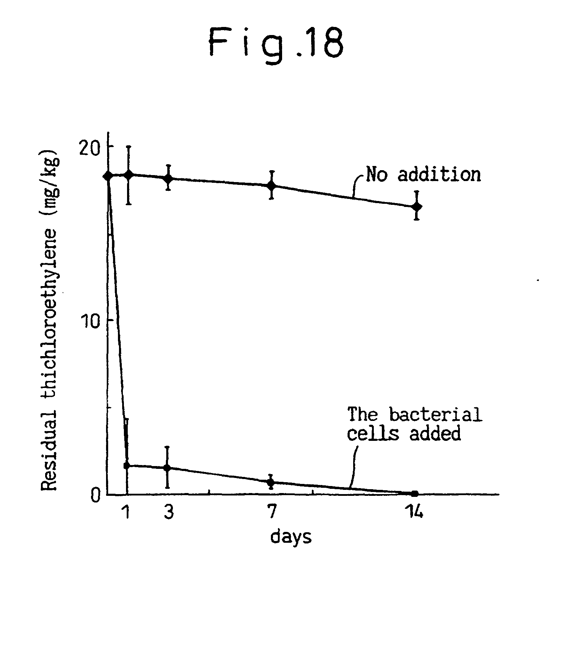

After the sample liquid was allowed to stand for 30 minutes, it was aspirated into a glass syringe. A filter holder fitted with a membrane filter having a pore size of 0.45 µ was connected to the syringe and the plunger was pressed to exclude the air and the initial few ml of the liquid, and then the filtrate was collected in fractions into stoppered test tubes, from which an amount necessary for determination was weighed out and was subjected to analysis by gaschromatography. The result as shown in Fig. 18 indicated that 90% of the total trichloroethylene in the soil was decomposed in a day after addition of the bacterial mass.

It was revealed therefore that the method of purifying trichloroethylene by adding the microorganism of the present invention into the soil is a technology which has a high purification ability almost equal to decomposition capacity of about 18 mg/kg of trichloroethylene in the soil in a day. When the soil sample at 7 days of standing was measured according to an analytical method designated by the nation (the Environmental Standard, the Soil Environmental Standard related to the contamination of the soil), the concentration of trichloroethylene was below the base value (0.03 ppm). As a result, it was revealed that trichloroethylene can be purified to the level which satisfies the environmental standard by purifying the contaminated soil using the microorganism of the present invention. It is generally accepted that the physical means of treatment such as vacuum extraction is unable to purify from low concentrations, but the use of the microorganism of the present invention enables purification to the level lower than the base value.

### Example 13.

To a 100 ml vial which can be sealed with a Teflon-coated rubber septum was added 30 g of sandy soil (air-dried) which was artificially contaminated with trichloroethylene. The microorganism of the present invention was cultured under shaking at 30°C for 3 days in the NMS medium which had 500 ppm of phenol and 0.05% yeast extract added thereto, and then was centrifuged to harvest the cells, which were resuspended into an appropriate amount of the NMS medium so that the water content in the soil became 25% and the inoculated amount of bacterial mass became 10⁸ to 10⁹ cells/g wet soil after addition of the suspension of the bacterial mass. After adding the suspension (containing no phenol) of the bacterial mass to the soil, the vial was sealed with a Teflon-coated rubber stopper, and then allowed to stand at 30°C for a certain period of time. Thereafter, analysis was conducted on byproducts which are said to be formed in association with trichloroethylene decomposition in the soil.

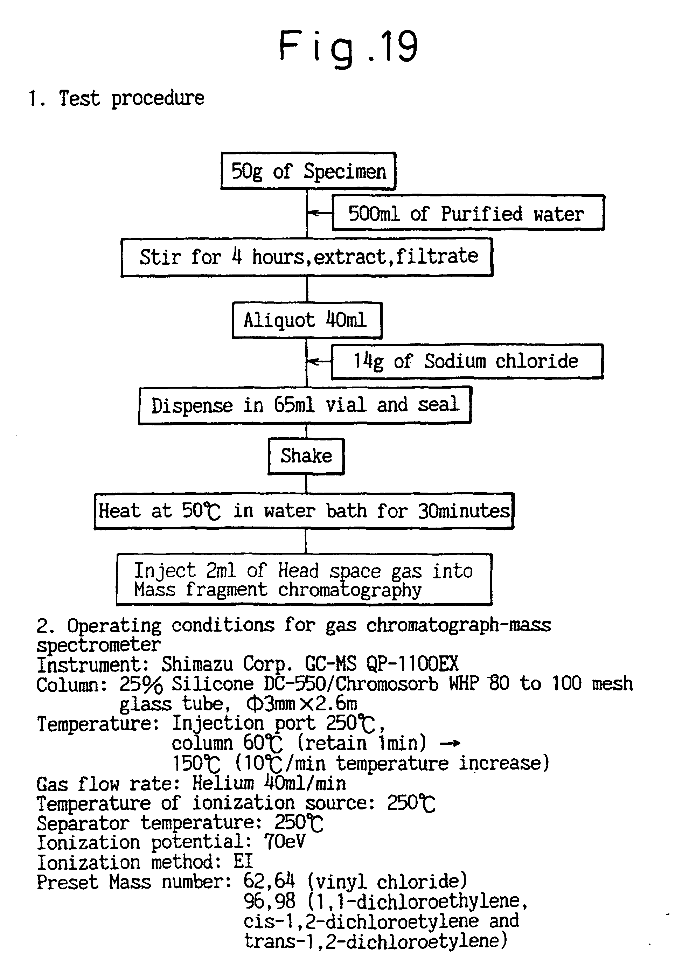

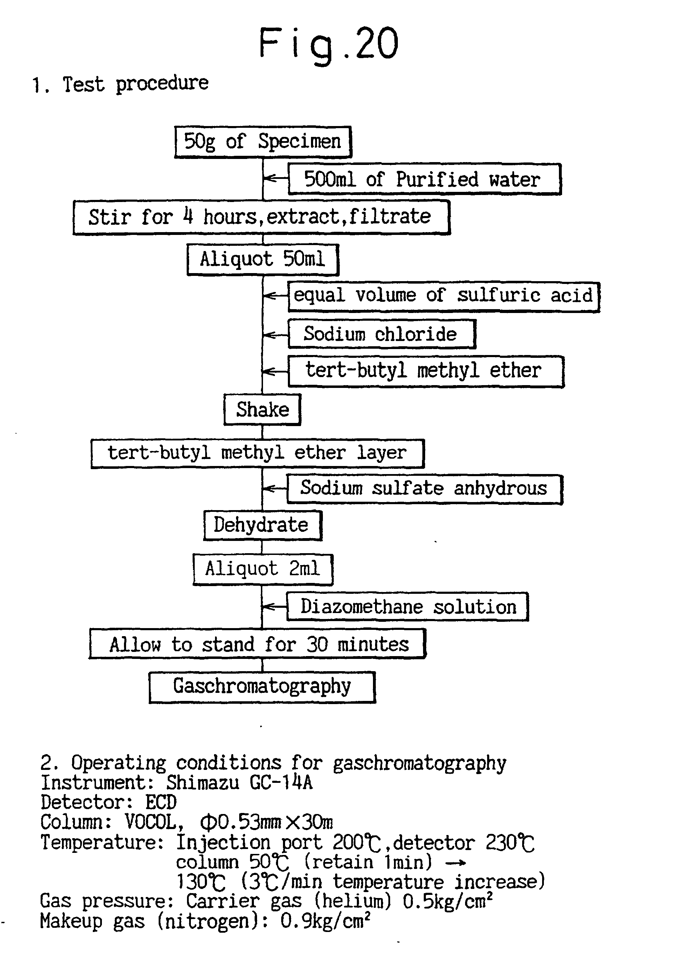

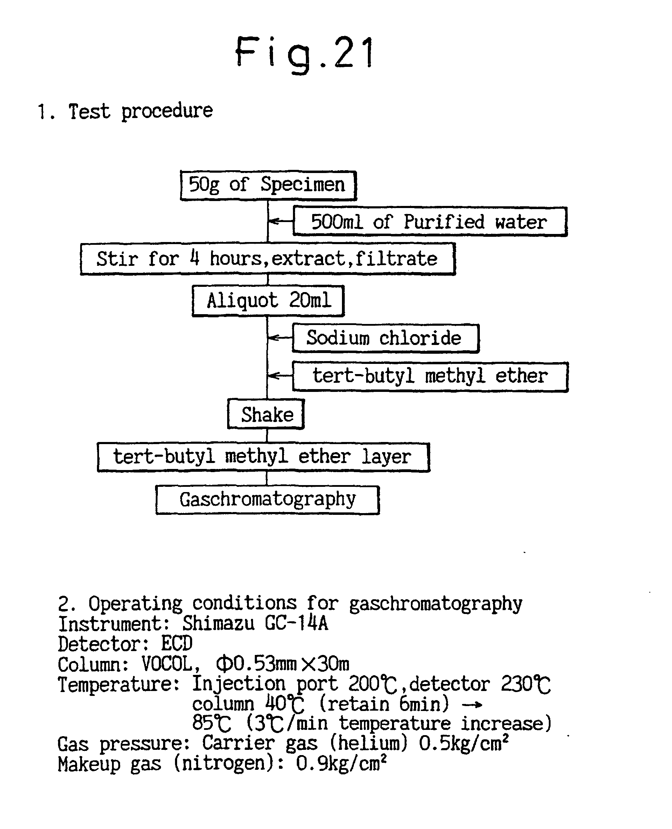

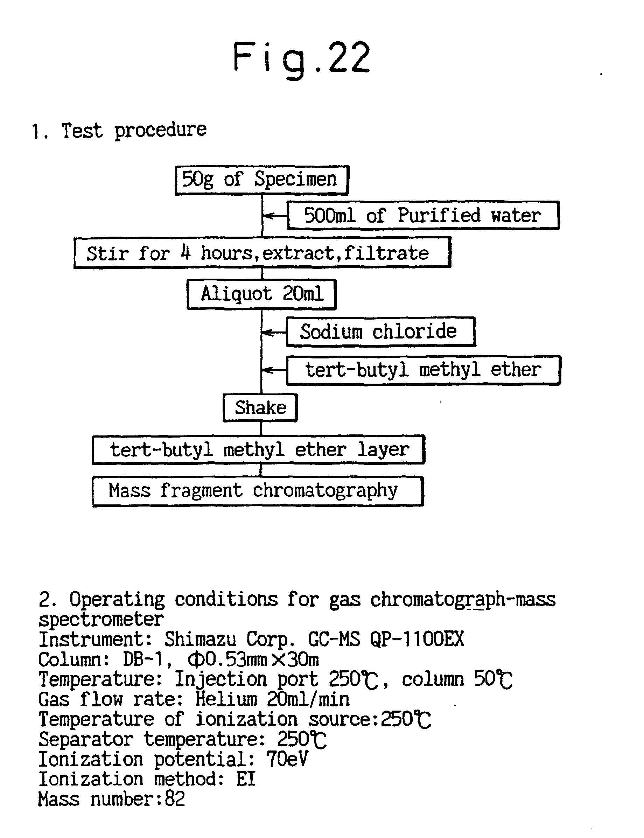

The byproducts in the soil were analyzed as follows: vinyl chloride, 1,1-dichloroethylene, cis-1,2-dichloroethylene, and trans-1,2-dichloroethylene were analyzed in the method shown in Fig. 19. Dichloro acetate and trichloro acetate, trichloroethanol, and hydrated chloral were analyzed in the method shown in Fig. 20, 21, and 22, respectively. The result as shown in Table 9 indicated that all byproducts were below the detection limit.

The dechlorination reaction of trichloroethylene under an anaerobic condition leads to accumulation of dichloroethylenes which is more toxic. It is also known that the decomposition process under an aerobic condition starting with formation of trichloroethylene oxide produces dichloro acetate etc. as intermediate products. There was no accumulation of harmful substances on decomposition of trichloroethylene by the microorganism of the present invention in the soil. Although the presence of substances other than those measured is unknown, this technology appeared to be a highly safe method.

**TABLE 8**

| Item to be analyzed | Result | Detection limit |
|---|---|---|
| Vinyl chloride | Not detected | 0.01 ppm |
| 1,1-dichloroethylene | Not detected | 0.01 ppm |
| cis-1,2-dichloroethylene | Not detected | 0.01 ppm |
| trans-1,2-dichloroethylene | Not detected | 0.01 ppm |
| Dichloroacetate | Not detected | 0.05 ppm |
| Trichloroethanol | Not detected | 0.01 ppm |
| Trichloroacetate | Not detected | 0.05 ppm |
| Hydrated chloral | Not detected | 0.05 ppm |

### Example 14.

To a 100 ml vial which can be sealed with a Teflon-coated rubber septum was added 30 g of sandy soil (air-dried) which was artificially contaminated with trichloroethylene. The microorganism of the present invention was cultured under shaking at 30°C for 3 days in the NMS medium which had 500 ppm of phenol and 0.05% yeast extract added thereto, and then was centrifuged to harvest the cells, which were resuspended into an appropriate amount of the NMS medium so that the water content in the soil became 25% and the inoculated amount of bacterial mass became 10⁸ to 10⁹ cells/g wet soil after addition of the suspension of the bacterial mass. After plating the suspension of the bacterial mass onto a petri dish to a thickness of about 1 mm, it was sterilized under irradiation of a 15 W Ultra violet lamp with a wavelength of 260 nm for not less than 60 seconds at a distance of 40 cm from the light source. After adding the suspension (containing no phenol) of the microorganism of the present invention into the soil, the vial was sealed with a Teflon-coated rubber stopper, and then allowed to stand at 30°C for a certain period of time.

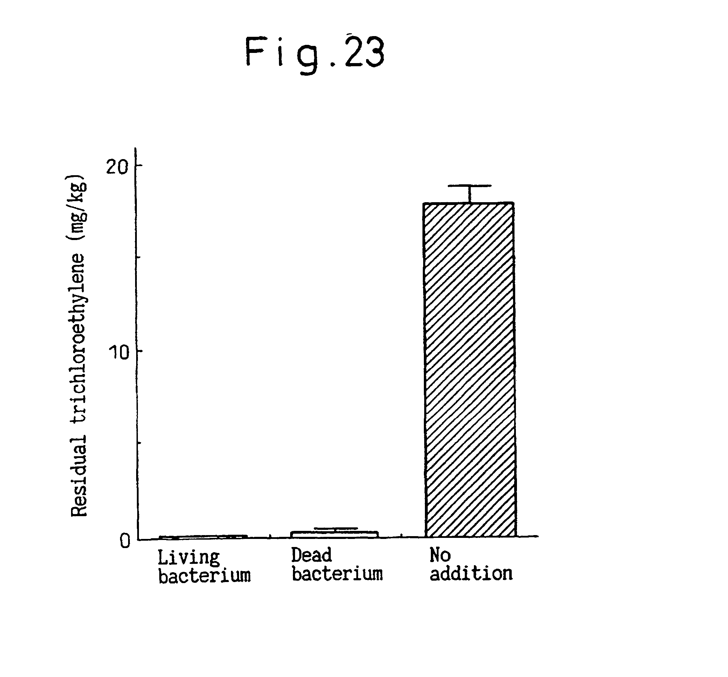

Thereafter, to a vial containing 30 g of the soil were added 50 ml of deionized water aerated with the air which had been passed through activated charcoal and 10 ml of n-hexane, and then the vial was sealed. The vial was sonicated in the sonicating washer for 10 minutes and then was shaken in a shaker for 10 minutes. The separated n-hexane was analyzed using a gaschromatograph equipped with an ECD detector. Furthermore, in an experiment in which the suspension of sterilized bacterial mass at an amount of 1/100 of that of the NYG medium was inoculated or the suspension was plated as it was onto the NYG agar medium and cultured at 30°C, neither increase in turbidity of the culture liquid nor colony formation were observed, and thereby it was confirmed that there was complete sterilization. The result as shown in Fig. 23 indicated that the dead cells and living cells gave an equal degree of decomposition of trichloroethylene.

Although most of the purification methods using microorganisms comprise adding living organisms into the soil, addition of the bacterial mass into the soil is currently difficult from a viewpoint of public acceptance. It is also being feared that it has a potential risk of producing a far-reaching effect on the ecological system by releasing a specific microorganism into the environment. But the addition of the microorganism which has completely lost the propagating activity by sterilization treatment is equivalent to that of mere organic materials, and thus is believed to have little effect on the ecological system. Therefore an experiment was conducted in which addition of the decomposing microorganism sterilized with ultraviolet irradiation into the soil was investigated. The result indicated that the addition of the dead bacterial mass proved to be an extremely useful method.

The invention disclosed in Japanese Unexamined Patent Publication No. 8(1996)-3012 claims that the effect on the ecological system can be minimized by crushing the decomposing bacteria and then spraying it to the soil. But, it is readily anticipated that the spraying of a large mass of decomposing bacterium to the contaminated soil is in fact difficult because crushing of microorganisms takes extensive equipment, a lot of time and labor. The known enzymes which have trichloroethylene-oxidizing activity require NAD as a coenzyme. But because the coenzyme is very expensive, it would be extremely difficult to supply the coenzyme in the concentration necessary for the decomposition. reaction of the enzyme which was released by crushing the bacterium from the bacterial mass. On the other hand, the method using the microorganism of the present invention has no such problems, and enables purification of trichloroethylene etc. at low cost and with minimum effects on the ecological system.

### Example 15.

To a 100 ml vial which can be sealed with a Teflon-coated rubber septum was added 30 g of sand grain soil (air-dried) which was artificially contaminated with trichloroethylene. The microorganism of the present invention was cultured under shaking at 30°C for 3 days in the NMS medium which had 500 ppm of phenol and 0.05% yeast extract added thereto, and then was centrifuged to harvest the cells, which were resuspended into an appropriate amount of the NMS medium so that the water content in the soil became 25% and the inoculated amount of bacterial mass became 10⁸ to 10⁹ cells/g wet soil after addition of the suspension of the bacterial mass. After plating the suspension of the bacterial mass onto a petri dish to a thickness of about 1 mm, and then sterilized under irradiation of a 15 W ultra violet lamp with a wavelength of 260 nm for not less than 60 seconds at a distance of 40 cm from the light source.

After adding the suspension (containing no phenol) of the sterilized microorganism of the present invention into the soil, the vial was sealed with a Teflon-coated rubber stopper, and then allowed to stand at 30°C for a certain period of time. After that, trichloroethylene in the soil was allowed to be decomposed and the byproducts which is believed to be formed in association with trichloroethylene decomposition were analyzed in a similar manner to Example 13. Furthermore, in an experiment in which the suspension of sterilized bacterial mass at an amount of 1/100 of that of the NYG medium was inoculated or the suspension was plated as it was onto the NYG agar medium and cultured at 30°C, neither increase in turbidity of the culture liquid nor colony formation were observed, and thereby it was confirmed that there was complete sterilization.

The result as shown in Table 10 indicated that all byproducts were below the detection limit. The dechlorination reaction of trichloroethylene under an anaerobic condition leads to accumulation of dichloroethylenes which is more toxic. It is also known that the decomposition process under an aerobic condition which starts with formation of trichloroethylene oxide produces dichloroacetate etc. as intermediate products. There was no accumulation of harmful substances on decomposition of trichloroethylene by the microorganism of the present invention in the soil. Although the presence of substances other than those measured is unknown, it was suggested that this technology is a highly safe method.

**TABLE 9**

| Item to be analyzed | Result | Detection limit |
|---|---|---|
| Vinyl chloride | Not detected | 0.01 ppm |
| 1,1-dichloroethylene | Not detected | 0.01 ppm |
| *cis*-1,2-dichloroethylene | Not detected | 0.01 ppm |
| trans-1,2-dichloroethylene | Not detected | 0.01 ppm |
| Dichloroacetate | Not detected | 0.05 ppm |
| Trichloroethanol | Not detected | 0.01 ppm |
| Trichloroacetate | Not detected | 0.05 ppm |
| Hydrated chloral | Not detected | 0.05 ppm |

### Example 16.

To a 100 ml vial which can be sealed with a Teflon-coated rubber septum was added 30 g of sandy soil (air-dried) which was artificially contaminated with trichloroethylene. The microorganism of the present invention was cultured under shaking at 30°C for 3 days in the NMS medium which had 500 ppm of phenol and 0.05% yeast extract added thereto, and then was centrifuged to harvest the cells, which were resuspended into an appropriate amount of the NMS medium so that the water content in the soil became 25% and the inoculated amount of bacterial mass was 10⁸ to 10⁹ cells/g wet soil after addition of the suspension of the bacterial mass. After adding the suspension (containing no phenol) of the microorganism of the present invention into the soil, the vial was sealed with a Teflon-coated rubber stopper, and then allowed to stand at 20°C for a certain period of time.

Thereafter, to a vial containing 30 g of the soil were added 50 ml of deionized water aerated with the air which had been passed through activated charcoal and 10 ml of n-hexane, and the vial was sealed. The vial was sonicated in the sonicating washer for 10 minutes and then was shaken in a shaker for 10 minutes. The separated n-hexane was analyzed using a gaschromatograph equipped with an ECD detector.

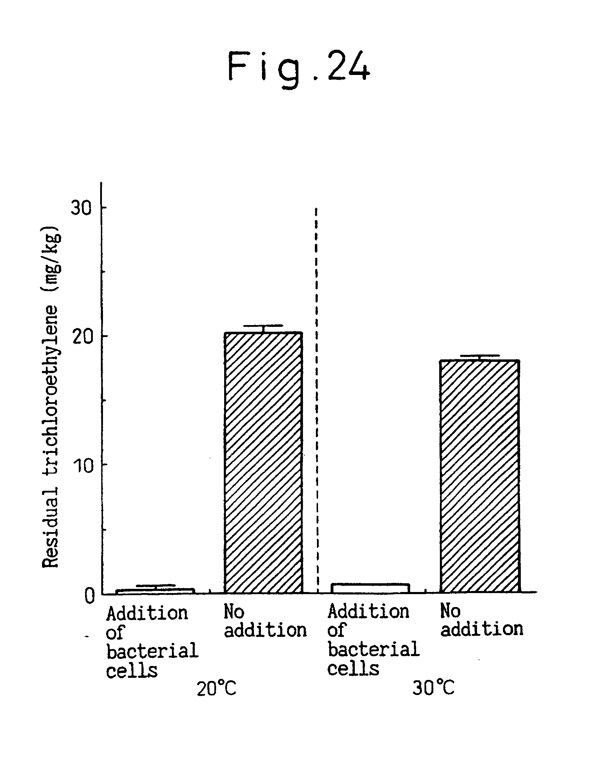

The result as shown in Fig. 24 indicated that trichloroethylene was decomposed at 20°C to a degree equivalent to that at 30°C. Since it was suggested that trichloroethylene is sufficiently decomposed at the temperature of the soil, the microorganism of the present invention proved to have a high practical utility as compared to the others.

### Example 17.

To a 100 ml vial was added 30 g of sand grain soil (air-dried) which was artificially contaminated with trichloroethylene. The microorganism of the present invention was cultured under shaking at 30°C for 1 day in the NMS medium which had 500 ppm of phenol and 0.05% yeast extract added thereto. The culture liquid at a volume of 1/100 of that of the NMS medium (or the PH medium) containing 500 ppm, 0.02%, and 1 mM of phenol, yeast extract, and glucose, respectively in the soil was added and then added to the soil. The density of the bacterial mass was set at 10⁶ cells/g wet soil, and the water content set at 25% at the time of culture liquid addition.

The vial was sealed with a Teflon-coated rubber septum, and then allowed to stand at 30°C for a certain period of time. Thereafter, to a vial containing 30 g of the soil were added 50 ml of deionized water aerated with the air which had been passed through activated charcoal and 10 ml of n-hexane, and then the vial was sealed. The vial was sonicated in the sonicating washer for 10 minutes and then was shaken in a shaker for 10 minutes. The separated n-hexane was analyzed using a gaschromatograph equipped with an ECD detector.

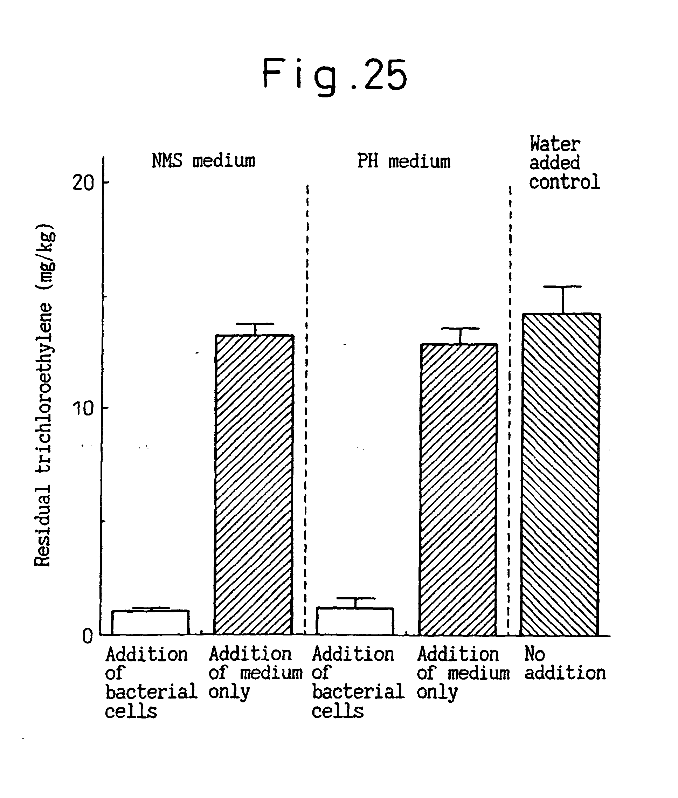

The result as shown in Fig. 25 indicated that trichloroethylene in the soil similarly decreased from about 14 mg/kg to about 1 mg/kg in the NMS medium and the PH medium. Due to the addition of phenol into the soil, autochthonous microorganisms contributed to reduction of trichloroethylene, but the result indicated a greater effect came from the addition of strain MO7. Therefore, it is clear that the possibility can be ruled out that the strain exerts its effect not only in the sterilized culture medium but also in the natural environment although autochthonous microorganisms are present. It was confirmed that the activity of strain MO7 is sufficiently induced by adding phenol at an amount of about 10⁶ cells/g wet soil even in the natural environment.

### Example 18. Construction of a constitutive mutant

A platinum loopful of the colony of the microorganism of the present invention which had been stored by passage on the agar plate containing 1.5% agar was picked and inoculated to a test tube containing 2 ml of the 1/3LB medium (its composition is shown in Table 11). After cultivating overnight under shaking at 30°C at 130 r.p.m., an aliquot of the culture broth was diluted as appropriate, which was then plated to the plate of the 1/3LB medium containing 1.5% agar. After cultivating at 30°C the number of cells was counted. The remainder of the culture broth was plated on a petri dish, which was then subjected to irradiation of a 15 W ultra violet lamp with a wavelength of 260 nm under the condition of a irradiation time of 3 minutes at a distance of 30 cm from the light source.

**TABLE 10**

| The 1/3LB Medium | |
|---|---|
| Tryptone | 3.0 g |
| Yeast extract | 1.5 g |
| Sodium chloride | 3.0 g |
| Distilled water | 1.0 liter |

Then, the culture broth was diluted as appropriate and was plated to the plate of the 1/3LB medium containing 1.5% agar. After culturing at 30°C the number of cells was counted to determine the death rate. When the death rate of 99% or higher was observed, it was assumed that there was enough mutation. From the culture broth at this point, the desired mutant were selected.

Catechol 2,3-dioxygenase (C230) introduces oxygen into catechol to form 2-hydroxymuconic acid semialdehyde by metafission. This product is yellow-colored. When C230 is expressed after being sprayed, it readily turns yellow. The trichloroethylene degrading enzymes of the phenol-utilizing trichloroethylene-decomposing bacteria are known to be the phenol hydroxylase (PH) which convert phenol into catechol (M. Fujita et al., J. Ferment. Bioeng., 79: 100, 1995; V. Shingler et al., J. Bacteriol., 174: 711, 1992).

An example has been known that C230 is expressed by one operon in which the C230 gene is adjacent to the PH gene though C230 itself was not directly involved in the decomposition of trichloroethylene (M. Fujita et al., J. Ferment. Bioeng., 79: 100, 1995; V. Shingler et al., J. Bacteriol., 174: 711, 1992). If C230 is expressed in association with the PH, it is possible to select the strains which express the trichloroethylene-degrading enzyme using C230 expression as an indicator. The microorganism of the present invention, when cultivated using phenol as the only carbon source, decomposes phenol and the culture broth turns yellow with the growth of the bacterium. This will make said selection method applicable to the microorganism.

Accordingly, the suspension of the bacterial mass after mutation was diluted as appropriate and was plated to the plate of the 1/3LB medium containing 1.5% agar. After cultivating at 30°C for 1 to 3 days, a catechol solution (0.1% (w/v) ethereal solution) was sprayed.

Then, when the death rate was 99.99%, about 10,000 colonies which appeared after ultra violet irradiation were investigated, which led to selection of 16 yellow colonies.

After the cononies were cultured in the 1/3LB liquid medium, it was plated to the plate of the 1/3LB medium containing 1.5% agar. After cultivating at 30°C, the colonies which appeared were sprayed with a catechol solution, and yellow colonies were isolated again to obtain the strains which consititutively express C23O.

- A platinum loopful of these strains which consititutively express C230 were picked and inoculated into the M medium (its composition is shown in Table 12) containing 4 ml of sodium glutamate (0.1%) in a 20 ml vial. The vial was plugged with a butyl rubber septum and sealed with an aluminum cap, which was then cultivated at 30°C under shaking for 2 days. Then trichloroethylene was added in an amount so that it became 30 ppm when all were dissolved in the liquid phase. After culturing at 30°C for 5 days, trichloroethylene concentration was determined as in Example 6.

**TABLE 11**

| M medium | |
|---|---|
| Ammonium nitrate | 3.0 g |
| Disodium hydrogen phosphate | 2.2 g |
| Potassium dihydrogen phosphate | 0.8 g |
| Ferrous sulfate (II) heptahydrate | 10.0 mg |
| Calcium sulfate dihydrate | 10.0 mg |
| Magnesium sulfate heptahydrate | 10.0 mg |
| Yeast extracts | 50.0 mg |
| Distilled water | 1.0 liter, pH 7.0 |

The result demonstrated that the parent strain M07 decomposed little trichloroethylene, but some of the strains which consititutively expressed C230 decomposed 52 to 34% of 30 ppm trichloroethylene. These microorganisms turned out to be a mutant which is derived from the parent strain MO7 and which can decompose trichloroethylene without requiring phenol.

Then, trichloroethylene decomposition in the soil is illustrated with reference to an example in which the strain MO715 which had the highest activity of decomposing trichloroethylene was used.

### Example 19. Decomposition of trichloroethylene bv a constitutive mutant

A soil which was artificially contaminated with trichloroethylene (11 mg/kg soil) was prepared as in Example 9. After cutivating the strain M0715 under shaking at 30°C for 2 days in 100 ml of the M medium containing sodium glutamate (0.1 w/v %), and then was centrifuged to harvest the cells, which were resuspended into an appropriate amount of the M medium so that the water content in the soil became 25%. After the suspension of the bacterial mass (containing no phenol) was added to the soil, the vial was sealed with a Teflon-coated rubber septum and was allowed to stand at 30°C for a certain period of time. Trichloroethylene was analyzed in a method similar to that in Example 9. The culture broth cultivated on phenol and the strain MO7 were also analyzed in a similar manner.

As a result, after the addition of the suspension of the bacterial mass of the strain M0715 cultivated with sodium glutamate, about 35% of trichloroethylene was decomposed in 12 hours and about 60% in 48 hours. The strain MO7 grown on sodium glutamate decomposed little trichloroethylene. Thus, it was revealed that the microorganism of the present invention does not require phenol for induction and can decompose trichloroethylene in the soil as well.

From the foregoin it can be concluded that the present invention is an extremely safe technology since trichloroethylene can be decomposed without spreading toxic substances such as phenol into the environment.

Reference to the international deposition of microorganisms under the Budapest Treaty

International Depository Authority: National Institute of Bioscience and Human-Technology Agency of Industrial Science and Technology

Address: 1-3, Higashi 1-chome, Tsukuba-shi, Ibaraki, 305, Japan

| Identification | Deposition Number | Deposition Date |
|---|---|---|
| MO7 | FERM BP-5624 | Aug. 12, 1996 |
| M0715 | FERM BP-5928 | Apl. 24, 1997 |

### SEQUENCE LISTING

SEQ ID No: 1
   Sequence length: 19
   Sequence type: nucleic acid
   Strandedness: single
   Topology: linear
   Molecule type: synthetic DNA
   Sequence description: GAGTTTGATC CTGGCTCAG
SEQ ID No: 2
   Sequence length: 27
   Sequence type: nucleic acid
   Strandedness: single
   Topology: linear
   Molecule type: synthetic DNA
   Sequence description: AGAAAGGAGG TGATCCAGCC GCAGGTT
SEQ ID No: 3
   Sequence length: 16
   Sequence type: nucleic acid
   Strandedness: single
   Topology: linear
   Molecule type: synthetic DNA
   Sequence description: GGCCGGACGG GTGAGT
SEQ ID No: 4
   Sequence length: 15
   Sequence type: nucleic acid
   Strandedness: single
   Topology: linear
   Molecule type: synthetic DNA
   Sequence description: TACGGAGGC AGCAG
SEQ ID No: 5
   Sequence length: 17
   Sequence type: nucleic acid
   Strandedness: single
   Topology: linear
   Molecule type: synthetic DNA
   Sequence description: GTGCCAGCAG CCGCGCG
SEQ ID No: 6
   Sequence length: 18
   Sequence type: nucleic acid
   Strandedness: single
   Topology: linear
   Molecule type: synthetic DNA
   Sequence description: GATTAGATAC CCTGGTAG
SEQ ID No: 7
   Sequence length: 18
   Sequence type: nucleic acid
   Strandedness: single
   Topology: linear
   Molecule type: synthetic DNA
   Sequence description: ACTCAAAGGA ATTGACGG
SEQ ID No: 8
   Sequence length: 16
   Sequence type: nucleic acid
   Strandedness: single
   Topology: linear
   Molecule type: synthetic DNA
   Sequence description: GCAACGAGCG CAACCC
SEQ ID No: 9
   Sequence length: 16
   Sequence type: nucleic acid
   Strandedness: single
   Topology: linear
   Molecule type: synthetic DNA
   Sequence description: TGTACACACC GCCCGT
SEQ ID No: 10
   Sequence length: 1422
   Sequence type: nucleic aci
   Strandedness: double
   Topology: linear
   Molecule type: cDNA
   Sequence description:

AACGCTGGCG GCGTGCTTAA CACATGCAAG TCGAACGGTG AAGCTTGGAG CTTGCTTCGA 60
GTGGATCAGT GGCGAACGGG TGAGTAACAC GTGAGCAACC TGCCCCAGAC TCTGGAATAA 120
GCGCTGGAAA CGGCGTCTAA TACTGGATAT GTGACGGACC TGCATGGGTA CCGTCTGGAA 180
AGTTTTTCGG TTTGGGATGG GCTCGCGGCC TATCAGCTTG TTGGTGAGGT AATGGCTCAC 240
CAAGGCGACA ACGGGTANCC GGCCTGAGAG GGCGACCGGC CACACTGGGA CTGAAACACG 300
GCCCAAACTC CTACGGGAGG CACCAGTGGG GAAATATTGC ACAATGGGCG AAAGCCTGAT 360
GCAGCGACGC CGCGTGAGGG ATGACGGCCT TCGGGTTGTA AACCTCTTTC AGCAGGGAAG 420
AAGCGAAAGT GACGGTACCT GCAGAATAAG CACCGGCTAA CTACGTGCCA GCAGCCGCGG 480
TAATACGTAG GGTGCGAGCG TTGTCCGGAA TTATTGGGCG TAAAGAGCTT GTAGGCGGTT 540
TGTCGCGTCT GCTGTGAAAA TCCGGGGCTC AACCCCGGAC TTGCAGTGGG TACGGGCAGA 600
CTAGAGTGTG GTAGGGGAGA CTGGAATTCC TGGTGTAGCG GTGAAATGCG CAGATATCAG 660
GAGGAACACC GATGGCGAAG GCAGGTCTCT GGGCCACTAC TGACGCTGAG AAGCGAAAGC 720
ATGGGGAGCG AACAGGATTA GATACCCTGG TAGTCCATGC CGTAAACGTT GGGCGCTAGG 780
TGTGGGACTC ATTCCACGAG TTCCGTGCCG CAGCTAACGC ATTAAGCGCC CCGCCTGGGG 840
CAGTACGGCC GCAAGGCTAA AACTCAAAGG AATTGACGGG GGCCCGCACA AGCGGCGGAG 900
CATGCGGATT AATTCGATGC AACGCGAAGA ACCTTACCAA GGCTTGACAT ATACCGGAAA 960
CTTCCAGAGA TGGTTGCCCC CTTTGGGTCG GTATACAGGT GGTGCATGGT TGTCGTCAGC 1020
TCGTGTCGTG AGATGTTGGG TTAAGTCCCG CAACGAGCGC AACCCTCGTT CTATGTTGCC 1080
AGCACGTCAT GGTGGGGACT CATGGAAGAC TGCCGGGGTC AACTCGGAAG AAGGTGGGGA 1140
TGACGTCAAA TCATCATGCC CCTTATGTCT TGGGCTTCAC GCATGCTACA ATGGCCGGTA 1200
CAAAGGGCTG CGATACCGCA AGGTGGAGCG AATCCCCAAA AAACCGGTCT CAGTTCGGAT 1260
TGGGGTCTGC AACTCGACCC CATGAAGTCG GAGTCGCTAG TAATCGCAAA TCAGCAACGC 1320

TGCGGTGAAT ACTTTCCCGG GCCTTGTACA CACCGCCCGT CAAGTCACGA AAGTTCGGTA 1380
ACACCCGAAG CCGGTGGCCC AACCCTTGTG GGGGGAGCCG TC 1422

## Claims

1. A bacterium which has following properties:
| | |
|---|---|
| Morphology | coccoid; rod shaped |
| Gram staining | + |
| Spore forming | - |
| Motility | - |
| Relationship to oxygen | aerobic |
| Oxidase test | - |
| Catalase test | + |
| Resistance to acid | - |
| Rod-coccus cycle | + |
| Aerial mycelium formation | - |
| Peptide glycan type of cell wall | meso-diaminopimelic acid |
| Glycolyl test | - (acetyl type) |
| Mycolic and GC content of DNA (mole%) | - 73 (by HPLC) |
which can decompose trichloroethylene, and which is strain MO7 (FERM BP-5624).

2. A method for decomposing organic halogenated compounds and/or aromatic compounds, comprising subjecting said organic halogenated compounds and/or aromatic compounds to the action of a bacterium according to claim 1.

3. A method according to claim 2 wherein the organic halogenated compound is trichloroethylene and the aromatic compound is a phenolic compound.

4. A method for decomposing organic halogenated compounds in the soil, waste waters, or other waste products containing organic halogenated compounds, comprising adding a culture of a bacterium according to claim 1 to the soil, waste waters, or other waste products.

5. A method according to claim 4 wherein the organic halogenated compound is trichloroethylene and the culture is a cultured bacterial cells.

6. A method according to claim 4 or 5 wherein an aromatic compound is added to a culture medium for use in culturing to obtain the bacterial culture.

7. A method according to claim 6 wherein said aromatic compound is a phenolic compound.

8. A method according to any of claims 4 to 7 wherein an degradable carbon source is added to a culture medium for use in culturing to obtain the bacterial culture.

9. A method according to claim 8 wherein the degradable carbon source is glucose.

10. A method according to any of claims 4 to 9 wherein the culture is cultured bacterial cells.

11. A method according to claim 10 wherein the cultured bacterial cells are living bacterial cells.

12. A method according to claim 10 wherein the cultured bacterial cells are sterilized cultured bacterial cells.

13. A method according to claim 12 wherein the sterilization treatment is ultraviolet irradiation.

14. A method for decomposing organic halogenated compounds in the soil, waste waters, or other waste products containing organic halogenated compounds, comprising the steps of inoculating a bacterium according to claim 1 to the soil, waste waters, or other waste products, adding an aromatic compound, an degradable carbon source, or a mixture thereof to the soil, waste waters, or other waste products, and then culturing said inoculated microorganism.

15. A method according to claim 14 wherein the organic halogenated compound is trichloroethylene.

16. A method according to claim 14 or 15 wherein the aromatic compound is a phenolic compound and the degradable carbon source is glucose.

17. A decomposition agent for organic halogenated compounds and/or aromatic compounds, comprising a culture of a bacterium according to claim 1.

18. A decomposition agent according to claim 17 wherein the culture is cultured bacterial cells.

19. A decomposition agent according to claim 18 wherein the culture is living bacterial cells.

20. A decomposition agent according to claim 18 wherein the cultured bacterial cells are sterilized bacterial cells.

21. A decomposition agent according to claim 20 wherein the sterilization treatment is ultraviolet irradiation.

22. A decomposition agent according to any of claims 17 to 21 which is in the form of dried bacterial cells.

23. A bacterium having the decomposition activity of organic halogenated compounds and/or aromatic compounds in the presence or absence of induction of an organic halogenated compound-decomposing enzyme by an aromatic compound, which bacterium is obtainable by subjecting a bacterium according to claim 1 to a mutation treatment and a selection procedure.

24. A bacterium according to claim 23 which is the strain MO715 (PERM BP-5928).

25. A method for decomposing organic halogenated compounds and/or aromatic compounds comprising allowing a bacterium according to claim 23 or 24 to act on the organic halogenated compounds and/or aromatic compounds.

26. A method according to claim 25 wherein the organic halogenated compound is trichloroethylene and the aromatic compound is a phenolic compound.

27. A method for decomposing organic halogenated compounds in the soil, waste waters, or other waste products containing organic halogenated compounds, comprising adding a culture of a bacterium according to claim 23 or 24 to the soil, waste waters, or other waste products.

28. A method according to claim 27 wherein the organic halogenated compound is trichloroethylene and the culture is cultured bacterial cells.

29. A method according to claim 27 or 28 wherein no aromatic compound is added to the culture medium for use in culturing to obtain said bacterial culture.

30. A method according to any of claims 27 to 29 wherein an degradable carbon source is added to the culture medium for use in culturing to obtain said bacterial culture.

31. A method according to claim 30 wherein the degradable carbon source is glutamic acid.

32. A method according to any of claims 27 to 31 wherein the culture is cultured bacterial cells.

33. A method according to claim 32 wherein the cultured bacterial cells are living bacterial cells.

34. A method according to claim 32 wherein the cultured bacterial cells are sterilized bacterial cells.

35. A method according to claim 34 wherein the sterilization treatment is ultraviolet irradiation.

36. A method for decomposing organic halogenated compounds in the soil, waste waters, or other waste products containing organic halogenated compounds, comprising the steps of inoculating bacterium according to claim 23 or 24 to the soil, waste waters, or other waste products, adding an degradable carbon source to said soil, waste waters, or other waste products, and then culturing the inoculated microorganism.

37. A method according to claim 36 wherein the organic halogenated compound is trichloroethylene.

38. A method according to claim 36 to 37 wherein the degradable carbon source is glutamic acid.

## Patentansprüche

1. Bakterium mit den folgenden Eigenschaften:
| | |
|---|---|
| Morphologie | kokkenähnlich; |
| | stäbchenförmig |
| Gramfärbung | + |
| Sporenbildung | - |
| Beweglichkeit | - |
| Verhältnis zu Sauerstoff | aerobisch |
| Oxidasetest | - |
| Katalasetest | + |
| Säurefestigkeit | - |
| Stäbchen-Kokkenzyklus | + |
| Luftmycelbildung | - |
| Peptidglykanart der Zellwand | Meso- |
| | Diaminopimelinsäure |
| Glycolyltest | - (Acetyltyp) |
| Mykolischer und GC-Gehalt der DNA (Mol-%) | - 73 (durch HPLC), |
welches Trichlorethylen abbauen kann, und welches der Stamm MO7 (FERM BP-5624) ist.

2. Verfahren für den Abbau organischer, halogenierter Verbindungen und/oder aromatischer Verbindungen, mit Aussetzen der organischen halogenierten Verbindungen und/oder aromatischen Verbindungen mit der Wirkung eines Bakteriums nach Anspruch 1.

3. Verfahren nach Anspruch 2, wobei die organische halogenierte Verbindung Trichlorethylen und die aromatische Verbindung eine phenolische Verbindung ist.

4. Verfahren für den Abbau von organischen, halogenierten Verbindungen im Boden, Abwässern oder anderen Abfallprodukten, die organische halogenierte Verbindungen enthalten, mit Zugabe einer Kultur eines Bakteriums nach Anspruch 1 zu dem Boden, den Abwässern oder anderen Abfallprodukten.

5. Verfahren nach Anspruch 4, wobei die organische halogenierte Verbindung Trichlorethylen ist, und die Kultur kultivierte Bakterienzellen ist.

6. Verfahren nach Anspruch 4 oder 5, wobei eine aromatische Verbindung zu einem Kulturmedium für die Verwendung in der Kultivierung zugegeben wird, um die Bakterienkultur zu erhalten.

7. Verfahren nach Anspruch 6, wobei die aromatische Verbindung eine phenolische Verbindung ist.

8. Verfahren nach einem der Ansprüche 4 bis 7, wobei eine abbaubare Kohlenstoffquelle zu einem Kulturmedium für die Verwendung in der Kultivierung zugegeben wird, um die Bakterienkultur zu erhalten.

9. Verfahren nach Anspruch 8, wobei die abbaubare Kohlenstoffquelle Glukose ist.

10. Verfahren nach einem der Ansprüche 4 bis 9, wobei die Kultur kultivierte Bakterienzellen ist.

11. Verfahren nach Anspruch 10, wobei die kultivierten Bakterienzellen lebende Bakterienzellen sind.

12. Verfahren nach Anspruch 10, wobei die kultivierten Bakterienzellen sterilisierte, kultivierte Bakterienzellen sind.

13. Verfahren nach Anspruch 12, wobei die Sterilisationsbehandlung ultraviolette Strahlung ist.

14. Verfahren für den Abbau organischer, halogenierter Verbindungen im Boden, Abwässern oder anderen Abfallprodukten, die organische halogenierte Verbindungen enthalten, mit den Schritten des Inokulierens eines Bakteriums nach Anspruch 1 in den Boden, die Abwässer oder andere Abfallprodukte, Zugabe einer aromatischen Verbindung, einer abbaubaren Kohlenstoffquelle oder einer Mischung davon zu dem Boden, den Abwässern oder anderen Abfallprodukten, und dann Kultivierung der inokulierten Mikroorganismen.

15. Verfahren nach Anspruch 14, wobei die organische halogenierte Verbindung Trichlorethylen ist.

16. Verfahren nach Anspruch 14 oder 15, wobei die aromatische Verbindung eine phenolische Verbindung ist, und die abbaubare Kohlenstoffquelle Glukose ist.

17. Abbaumittel für organische, halogenierte Verbindungen und/oder aromatische Verbindungen, mit einer Kultur eines Bakteriums nach Anspruch 1.

18. Abbaumittel nach Anspruch 17, wobei die Kultur kultivierte Bakterienzellen ist.

19. Abbaumittel nach Anspruch 18, wobei die Kultur lebende Bakterienzellen ist.

20. Abbaumittel nach Anspruch 18, wobei die kultivierten Bakterienzellen sterilisierte Bakterienzellen sind.

21. Abbaumittel nach Anspruch 20, wobei die Sterilisationsbehandlung ultraviolette Bestrahlung ist.

22. Abbaumittel nach einem der Ansprüche 17 bis 21, welches in der Form von getrockneten Bakterienzellen ist.

23. Bakterium mit Abbauaktivität für organische, halogenierte Verbindungen und/oder aromatische Verbindungen in der Anwesenheit oder Abwesenheit der Induktion eines Enzyms, das organische halogenierte Verbindungen abbaut, durch eine aromatische Verbindung, wobei das Bakterium durch Unterziehen des Bakteriums nach Anspruch 1 mit einer Mutationsbehandlung und einem Selektionsverfahren erhältlich ist.

24. Bakterium nach Anspruch 23, welches der Stamm MO715 (FERM BP-5928) ist.

25. Verfahren für den Abbau organischer, halogenierter Verbindungen und/oder aromatischer Verbindungen mit Wirkenlassen des Bakteriums nach Anspruch 23 oder 24 auf organische halogenierte Verbindungen und/oder aromatische Verbindungen.

26. Verfahren nach Anspruch 25, wobei die organische, halogenierte Verbindung Trichlorethylen und die aromatische Verbindung eine phenolische Verbindung ist.

27. Verfahren für den Abbau organischer, halogenierter Verbindungen im Boden, Abwässern oder anderen Abfallprodukten, die organische halogenierte Verbindungen enthalten, mit Zugabe einer Kultur eines Bakteriums nach Anspruch 23 oder 24 zum Boden, den Abwässern oder den anderen Abfallprodukten.

28. Verfahren nach Anspruch 27, wobei die organische, halogenierte Verbindung Trichlorethylen ist und die Kultur kultivierte Bakterienzellen ist.

29. Verfahren nach Anspruch 27 oder 28, wobei keine aromatische Verbindung zu dem Kulturmedium für die Verwendung in der Kultivierung zugegeben wird, um die Bakterienkultur zu erhalten.

30. Verfahren nach einem der Ansprüche 27 bis 29, wobei eine abbaubare Kohlenstoffquelle zu dem Medium für die Verwendung in der Kultivierung zugegeben wird, um die Bakterienkultur zu erhalten.

31. Verfahren nach Anspruch 30, wobei die abbaubare Kohlenstoffquelle Glutaminsäure ist.

32. Verfahren nach einem der Ansprüche 27 bis 31, wobei die Kultur kultivierte Bakterienzellen ist.

33. Verfahren nach Anspruch 32, wobei die kultivierten Bakterienzellen lebende Bakterienzellen sind.

34. Verfahren nach Anspruch 32, wobei die kultivierten Bakterienzellen sterilisierte Bakterienzellen sind.

35. Verfahren nach Anspruch 34, wobei die Sterilisationsbehandlung ultraviolette Bestrahlung ist.

36. Verfahren für den Abbau organischer halogenierter Verbindungen im Boden, Abwässern oder anderen Abfallprodukten, die organische halogenierte Verbindungen enthalten, mit den Schritten der Inokulierung des Bakteriums nach Anspruch 23 oder 24 in den Boden, die Abwässer oder die anderen Abfallprodukte, Zugabe einer abbaubaren Kohlenstoffquelle zu dem Boden, den Abwässern oder den anderen Abfallprodukten, und dann Kultivierung der inokulierten Mikroorganismen.

37. Verfahren nach Anspruch 36, wobei die organische, halogenierte Verbindung Trichlorethylen ist.

38. Verfahren nach Anspruch 36 bis 37, wobei die abbaubare Kohlenstoffquelle Glutaminsäure ist.

## Revendications

1. Bactérie qui possède les propriétés suivantes :
| | |
|---|---|
| morphologie | coccoïde ; en forme de bâtonnet |
| coloration de Gram | + |
| formation de spores | - |
| motilité | - |
| relation à l'oxygène | aérobie |
| test à l'oxydase | - |
| test à la catalase | + |
| résistance aux acides | - |
| cycle bâtonnet-coccoïde | + |
| formation de mycélium aérien type de peptidoglycane de | - |
| la paroi cellulaire diaminopimélique | acide méso- |
| test au glycolyle | - (type acétyle) |
| teneur en acide mycolique et en GC de l'ADN (% molaire) | - 73 (par CLHP) |
qui peut décomposer le trichloréthylène, et qui constitue la souche MO7 (FERM BP-5624).

2. Procédé de décomposition de composés organiques halogénés et/ou de composés aromatiques, consistant à soumettre lesdits composés organiques halogénés et/ou composés aromatiques à l'action d'une bactérie selon la revendication 1.

3. Procédé selon la revendication 2 dans lequel le composé organique halogéné est le trichloréthylène et le composé aromatique est un composé phénolique.

4. Procédé de décomposition de composés organiques halogénés dans le sol, les eaux usées ou d'autres déchets contenant des composés organiques halogénés, consistant à ajouter une culture d'une bactérie selon la revendication 1 au sol, aux eaux usées ou aux autres déchets.

5. Procédé selon la revendication 4 dans lequel le composé organique halogéné est le trichloréthylène et la culture consiste en des cellules bactériennes cultivées.

6. Procédé selon la revendication 4 ou 5 dans lequel un composé aromatique est ajouté à un milieu de culture à utiliser en culture pour obtenir la culture bactérienne.

7. Procédé selon la revendication 6 dans lequel ledit composé aromatique est un composé phénolique.

8. Procédé selon l'une quelconque des revendications 4 à 7 dans lequel une source de carbone dégradable est ajoutée à un milieu de culture à utiliser en culture pour obtenir la culture bactérienne.

9. Procédé selon la revendication 8 dans lequel la source de carbone dégradable est le glucose.

10. Procédé selon l'une quelconque des revendications 4 à 9 dans lequel la culture consiste en des cellules bactériennes cultivées.

11. Procédé selon la revendication 10 dans lequel les cellules bactériennes cultivées sont des cellules bactériennes vivantes.

12. Procédé selon la revendication 10 dans lequel les cellules bactériennes cultivées sont des cellules bactériennes cultivées stérilisées.

13. Procédé selon la revendication 12 dans lequel le traitement de stérilisation est une irradiation aux ultraviolets.

14. Procédé de décomposition de composés organiques halogénés dans le sol, les eaux usées ou d'autres déchets contenant des composés organiques halogénés, comprenant les étapes d'inoculation d'une bactérie selon la revendication 1 dans le sol, les eaux usées ou les autres déchets, d'addition d'un composé aromatique, d'une source de carbone dégradable ou d'un mélange de ceux-ci au sol, aux eaux usées ou aux autres déchets, puis de culture dudit micro-organisme inoculé.

15. Procédé selon la revendication 14 dans lequel le composé organique halogéné est le trichloréthylène.

16. Procédé selon la revendication 14 ou 15 dans lequel le composé aromatique est un composé phénolique et la source de carbone dégradable est le glucose.

17. Agent de décomposition pour composés organiques halogénés et/ou composés aromatiques, comprenant une culture d'une bactérie selon la revendication 1.

18. Agent de décomposition selon la revendication 17 dans lequel la culture consiste en des cellules bactériennes cultivées.

19. Agent de décomposition selon la revendication 18 dans lequel la culture consiste en des cellules bactériennes vivantes.

20. Agent de décomposition selon la revendication 18 dans lequel les cellules bactériennes cultivées sont des cellules bactériennes stérilisées.

21. Agent de décomposition selon la revendication 20 dans lequel le traitement de stérilisation est une irradiation aux ultraviolets.

22. Agent de décomposition selon l'une quelconque des revendications 17 à 21 qui est sous la forme de cellules bactériennes séchées.

23. Bactérie possédant une activité de décomposition de composés organiques halogénés et/ou de composés aromatiques en présence ou en l'absence d'induction d'une enzyme de décomposition de composés organiques halogénés par un composé aromatique, laquelle bactérie peut être obtenue en soumettant une bactérie selon la revendication 1 à un traitement mutationnel et à une procédure de sélection.

24. Bactérie selon la revendication 23 qui constitue la souche MO715 (FERM BP-5928).

25. Procédé de décomposition de composés organiques halogénés et/ou de composés aromatiques consistant à laisser une bactérie selon la revendication 23 ou 24 agir sur les composés organiques halogénés et/ou les composés aromatiques.

26. Procédé selon la revendication 25 dans lequel le composé organique halogéné est le trichloréthylène et le composé aromatique est un composé phénolique.

27. Procédé de décomposition de composés organiques halogénés dans le sol, les eaux usées ou d'autres déchets contenant des composés organiques halogénés, consistant à ajouter une culture d'une bactérie selon la revendication 23 ou 24 au sol, aux eaux usées ou aux autres déchets.

28. Procédé selon la revendication 27 dans lequel le composé organique halogéné est le trichloréthylène et la culture consiste en des cellules bactériennes cultivées.

29. Procédé selon la revendication 27 ou 28 dans lequel aucun composé aromatique n'est ajouté au milieu de culture à utiliser en culture pour obtenir ladite culture bactérienne.

30. Procédé selon l'une quelconque des revendications 27 à 29 dans lequel une source de carbone dégradable est ajoutée au milieu de culture à utiliser en culture pour obtenir ladite culture bactérienne.

31. Procédé selon la revendication 30 dans lequel la source de carbone dégradable est l'acide glutamique.

32. Procédé selon l'une quelconque des revendications 27 à 31 dans lequel la culture consiste en des cellules bactériennes cultivées.

33. Procédé selon la revendication 32 dans lequel les cellules bactériennes cultivées sont des cellules bactériennes vivantes.

34. Procédé selon la revendication 32 dans lequel les cellules bactériennes cultivées sont des cellules bactériennes stérilisées.

35. Procédé selon la revendication 34 dans lequel le traitement de stérilisation est une irradiation aux ultraviolets.

36. Procédé de décomposition de composés organiques halogénés dans le sol, les eaux usées ou d'autres déchets contenant des composés organiques halogénés, comprenant les étapes d'inoculation d'une bactérie selon la revendication 23 ou 24 au sol, aux eaux usées ou aux autres déchets, d'addition d'une source de carbone dégradable auxdits sol, eaux usées ou autres déchets, puis de culture du micro-organisme inoculé.

37. Procédé selon la revendication 36 dans lequel le composé organique halogéné est le trichloréthylène.

38. Procédé selon la revendication 36 ou 37 dans lequel la source de carbone dégradable est l'acide glutamique.
